# EUROPEAN PATENT APPLICATION

(11) **EP 3 254 637 A1**
(43) Date of publication of application: **13.12.2017**
(21) Application number: 15881202.4
(22) Date of filing: 08.12.2015
(51) Int. Cl.: A61B 90/00, A61B 1/00

(54) **MEDICAL MANIPULATOR SYSTEM**

(30) Priority: 06.02.2015 JP 2015022614
(71) Applicant: Olympus Corporation, Hachioji-shi Tokyo 192-8507 (JP)
(72) Inventor: YOSHII, Toshihiro, Tokyo 192-8507 (JP)
(74) Representative: Schicker, Silvia
(86) International application number: PCT/JP2015/084371
(87) International publication number: WO 2016/125385

(57) **Abstract**

A medical manipulator system (1) includes a guide instrument having a treatment tool channel, an insertion body (31) inserted into the treatment tool channel, a power source unit (56) attachable to and detachable from the insertion body (31), a guide rail (53) having a first region which corresponds to a state in which the joint part of the insertion body (31) is located in the treatment tool channel and a second region which corresponds to a state in which the joint part is exposed from the distal end of the treatment tool channel, and the guide rail is configured to guide the power source unit (56) to advance and retract in a predetermined linear direction including the first region and the second region, and a regulating part (70) interposed in a boundary between the first region and the second region in an advancing/retracting path of the power source unit (56) in the guide rail (53) and the regulating part is configured to regulate a movement of the insertion body (31) from the second region toward the first region in a state in which the insertion body (31) is attached to the power source unit (56).

## Description

### [Technical Field]

The present invention relates to a medical manipulator system.

Priority is claimed on Japanese Patent Application No. 2015-022614, filed on February 6, 2015, the content of which is incorporated herein by reference.

### [Background Art]

Conventionally, in a surgical operation or the like using a medical manipulator system including an endoscope, by advancing and retracting a treatment tool with respect to a treatment tool channel of the endoscope, a treatment tool is accommodated in the treatment tool channel or the treatment tool is protruded from inside of the treatment tool channel toward a treatment target.

For example, in Patent Literature 1, a treatment tool advance-and-retract regulating mechanism connected to an inlet of a treatment tool channel of an endoscope is disclosed. The treatment tool advance-and-retract regulating mechanism described in Patent Literature 1 regulates advancing and retracting amounts of a distal end part of a treatment tool within a fixed range between a predetermined position in the vicinity of a distal interior of a treatment tool channel and a distal exterior of the treatment tool channel. In the technology disclosed in Patent Literature 1, it is possible to set an initial position of a treatment tool at a predetermined position in the treatment tool channel of the endoscope and to cause the treatment tool to protrude from the treatment tool channel after the initial position is set.

For example, in Patent Literature 2, a medical instrument capable of advancing and retracting a treatment tool having a bendable distal end part in an elongated shaft and projecting and retracting the distal end part of the treatment tool from and into the elongated shaft is disclosed.

### [Patent Literature]

[Patent Literature 1] Japanese Unexamined Patent Application, First Publication No. 2010-69003
[Patent Literature 2] United States Patent No. 7854738

### [Summary of Invention]

### [Technical Problem]

When a treatment tool having a bendable distal end part is drawn into a treatment tool channel, it is preferable that the distal end of the treatment tool follows the treatment tool channel such that the treatment tool is smoothly drawn in to the treatment tool channel. However, in the technology disclosed in Patent Literatures 1 and 2, the possibility that the treatment tool is drawn into the treatment tool channel by an erroneous operation in a state in which the distal end part of the treatment tool is bent is not taken into consideration.

The present invention has been made in view of the above-described problems, and an objective of the present invention is to provide a medical manipulator system capable of preventing a treatment tool from being drawn into a treatment tool channel by an erroneous operation in a state in which a distal end part of the treatment tool is bent.

### [Solution to Problem]

According to an aspect of the present invention, a medical manipulator system includes a guide instrument that has a treatment tool channel and the guide instrument is inserted into a body, an insertion body having a joint part that is bendable by receiving predetermined power and the insertion body is inserted into the treatment tool channel, a power source unit attachable to and detachable from the insertion body and the power source unit is configured to transmit the power to the insertion body, a guide rail having a first region which corresponds to a state in which the joint part is located in the treatment tool channel and a second region which is connected to the first region to correspond to a state in which the joint part is exposed from a distal end of the treatment tool channel, and the guide rail is configured to advance and retract the insertion body in the treatment tool channel by guiding the power source unit to advance and retract in a predetermined linear direction defined by the first region and the second region, and a regulating part interposed in a boundary between the first region and the second region in an advancing/retracting path of the power source unit in the guide rail and the regulating part is configured to regulate a movement of the insertion body from the second region toward the first region in a state in which the insertion body and the power source unit are attached such that the power is able to be transmitted.

The regulating part may include an opening/closing member disposed on a movement path of the insertion body along the guide rail in a state in which the insertion body is attached to the power source unit to come into contact with at least part of the insertion body and the power source unit, a support body configured to support the opening/closing member at the boundary such that the opening/closing member is able to be inserted into and removed from the movement path, and a biasing member configured to bias the opening/closing member toward the support body such that the opening/closing member is disposed on the movement path. The opening/closing member may include a first surface facing the first region and the first surface is able to come into contact with at least part of the insertion body and the power source unit, and a second surface facing the second region and the second surface is able to abut at least part of the insertion body and the power source unit in a direction of the movement path. The insertion body may come into contact with the first surface to generate a space for introducing the insertion body into the second region by pushing the opening/closing member out of the movement path against a biasing force of the biasing member, in a state in which the insertion body is attached to the power source unit during a process in which the insertion body moves from the first region to the second region along the movement path.

The first surface may be a tapered surface tilted with respect to the direction of the movement path. At least part of the insertion body and the power source unit may generate a space for introducing the insertion body into the second region by coming into contact with the tapered surface to push the opening/closing member out of the movement path against a biasing force of the biasing member, in a state in which the insertion body is attached to the power source unit during a process in which the insertion body moves from the first region to the second region.

The opening/closing member may be supported by the support body such that the opening/closing member is rotatable about a rotation axis extending in a direction orthogonal to the direction of the movement path.

The medical manipulator system according to the above aspect may further include a returning mechanism configured to move the power source unit from the second region toward the first region along the guide rail.

The medical manipulator system according to the above aspect may further include an attachment/detachment-regulating means configured to prevent an attachment of the insertion body to the power source unit disposed in the second region, and the attachment/detachment-regulating means may be configured to allow a detachment of the insertion body from the power source unit when the power source unit disposed in the second region has already been attached to the insertion body.

The medical manipulator system according to the above aspect may further include a mounting sensor capable of detecting that the insertion body is attached to the power source unit, a position sensor capable of detecting which of the first region and the second region the power source unit is located in, and a control unit configured to control the regulating part to regulate movement of the power source unit between the first region and the second region when the mounting sensor detects that the insertion body is attached to the power source unit and the position sensor detects that the power source unit is in the second region.

In the medical manipulator system according to the above aspect, the control unit may cancel the control of the regulation by the regulating part and enable the power source unit to be movable from the second region to the first region, when the mounting sensor detects that the insertion body is not attached to the power source unit and the position sensor detects that the power source unit is in the second region.

### [Advantageous Effects of Invention]

According to the present invention, it is possible to prevent a treatment tool from being drawn into a treatment tool channel by an erroneous operation in a state in which a distal end part of the treatment tool is bent.

### [Brief Description of Drawings]

Fig. 1 is an overall view of a medical manipulator system according to a first embodiment of the present invention.
Fig. 2 is a schematic diagram showing a surgical tool of the medical manipulator system.
Fig. 3 is a partial cross-sectional view showing a guide instrument of the surgical tool.
Fig. 4 is a schematic diagram showing a treatment tool of the surgical tool.
Fig. 5 is an enlarged partial cross-sectional view showing a distal end part of an insertion body of the treatment tool.
Fig. 6 is a partial cross-sectional view showing an attachable/detachable part of the insertion body.
Fig. 7 is a side view showing a driver body of the treatment tool.
Fig. 8 is a rear view of the driver body.
Fig. 9 is a plan view of the driver body.
Fig. 10 is a partial cross-sectional view showing a process in which the insertion body is inserted into a treatment tool channel of the guide instrument.
Fig. 11 is a side view showing a process in which the attachable/detachable part of the insertion body moves along a guide rail of the driver body.
Fig. 12 is a rear view showing a process in which the attachable/detachable part of the insertion body moves along the guide rail of the driver body.
Fig. 13 is a side view showing a process in which the attachable/detachable part of the insertion body moves along the guide rail of the driver body.
Fig. 14 is a side view showing a process in which, after the insertion body is detached from a power source unit of the driver body, the power source unit moves.
Fig. 15 is a side view showing a configuration of a modified example of the embodiment.
Fig. 16 is a plan view showing the configuration of the modified example.
Fig. 17 is a side view showing a configuration of another modified example of the embodiment.
Fig. 18 is a plan view showing the configuration of the modified example.
Fig. 19 is a side view showing a treatment tool in a medical manipulator system according to a second embodiment of the present invention.
Fig. 20 is a rear view of the treatment tool.
Fig. 21 is a plan view of the treatment tool.
Fig. 22 is a block diagram showing a partial configuration of a slave control circuit of the medical manipulator system according to the embodiment.
Fig. 23 is a flowchart showing a part of processing in the slave control circuit.
Fig. 24 is a plan view showing a process in which a power source unit of a driver body moves along a guide rail of the driver body.
Fig. 25 is a cross-sectional view taken along line A-A of Fig. 24.
Fig. 26 is a side view showing a treatment tool in a medical manipulator system according to a third embodiment of the present invention.
Fig. 27 is a side view showing a configuration of a modified example of the embodiment.
Fig. 28 is a side view showing a treatment tool in a medical manipulator system according to a fourth embodiment of the present invention.
Fig. 29 is a plan view of the treatment tool.
Fig. 30 is a plan view showing a process in which a power source unit of a driver body moves along a guide rail of the driver body.
Fig. 31 is a partial cross-sectional view showing a treatment tool in a medical manipulator system according to a fifth embodiment of the present invention in a side view.
Fig. 32 is a view describing an action of the treatment tool of the embodiment.

### [Description of Embodiments]

### (First embodiment)

A first embodiment of the present invention will be described.

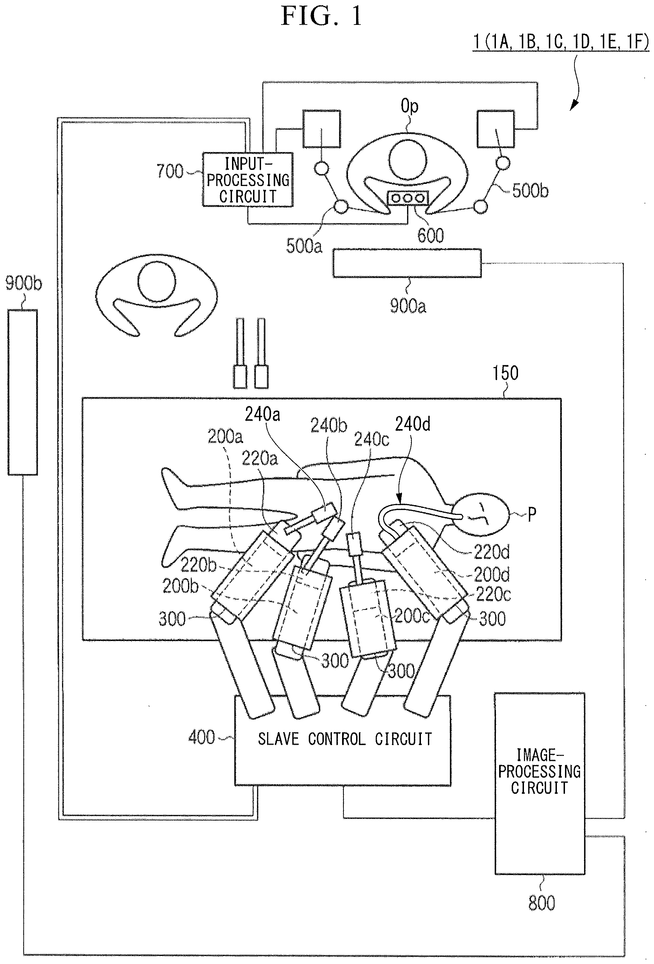

First, an overall schematic configuration of a medical manipulator system according to the present embodiment will be described. Fig. 1 is an overall view of a medical manipulator system according to a first embodiment of the present invention.

A medical manipulator system 1 of the present embodiment shown in Fig. 1 is a master-slave type manipulator system. The master-slave type medical manipulator system 1 is a system having two types of arms including a master arm and a slave arm and in which the slave arm is remotely controlled to follow a motion of the master arm.

The medical manipulator system 1 shown in Fig. 1 includes an operating table 150, slave arms 200a, 200b, 200c, and 200d, a slave control circuit 400, master arms 500a and 500b, an operation part 600, an input-processing circuit 700, an image-processing circuit 800, an operator display 900a, and an assistant display 900b.

Hereinafter, to make description concise, reference numerals "Xa, Xb ... Xz" in alphabetical order are written as "Xa to Xz" in some cases. For example, "slave arms 200a, 200b, 200c, 200d" is written as "slave arms 200a to 200d" in some cases.

The operating table 150 is a support table on which a patient P to be observed or treated is placed. A plurality of slave arms 200a to 200d are installed in the vicinity of the operating table 150. The slave arms 200a to 200d can also be installed at the operating table 150.

Each of the slave arms 200a to 200d has a plurality of multi-degree-of-freedom joints. Each of the slave arms 200a to 200d includes surgical tools 240a to 240d attachable to and detachable from the slave arms 200a to 200d, respectively.

The multi-degree-of-freedom joints of each of the slave arms 200a to 200d are bent to determine the positions of the surgical tools 240a to 240d with respect to the patient P placed on the operating table 150.

The surgical tools 240a to 240d are mounted at a distal end side of the slave arms 200a to 200d (defined to be a side facing a body cavity of the patient P).

Each of the multi-degree-of-freedom joints is separately driven by a power unit (not shown). As the power unit, for example, a motor having a servo mechanism which includes an incremental encoder, a decelerator, or the like may be used. Operation of the power unit configured to drive each of the multi-degree-of-freedom joints is controlled by the slave control circuit 400.

In FIG. 1, reference numerals 220a, 220b, 220c, and 220d represent surgical power transmission adapters. The surgical power transmission adapters 220a, 220b, 220c, and 220d are interposed between the slave arms 200a, 200b, 200c, and 200d and the surgical tools 240a, 240b, 240c, and 240d. The surgical power transmission adapters 220a, 220b, 220c, and 220d respectively connect the slave arms 200a, 200b, 200c, and 200d to the surgical tools 240a, 240b, 240c, and 240d.

The surgical tools 240a to 240d may be rigid or flexible. In other words, as the surgical tools 240a to 240d, a configuration in which an operating body such as a treatment part or a joint part for performing treatment on a living body is operated by pushing and pulling a rigid rod or a configuration in which an operating body which operates in the body is operated by towing a flexible wire to perform treatment on a living body can be appropriately selected and adopted. Even when the surgical tools 240a to 240d are rigid, a configuration in which the operating body is operated by towing a flexible wire is possible.

In Fig. 1, an example in which the surgical tools 240a to 240c are rigid and the surgical tool 240d is flexible is shown. The flexible surgical tool 240d is introduced into the body from a patient's natural opening such as the mouth and through the digestive canal and the like.

The slave control circuit 400 is configured to include, for example, a central processing unit (CPU), a memory, or the like. The slave control circuit 400 stores a predetermined program for controlling the slave arms 200a to 200d. The slave control circuit 400 controls operations of the slave arms 200a to 200d or the surgical tools 240a to 240d according to a control signal from the input-processing circuit 700. The slave control circuit 400 specifies a slave arm which is an operation target of a master arm operated by an operator Op on the basis of the control signal from the input-processing circuit 700, and calculates a driving amount necessary for causing the specified slave arm or the like to move corresponding to an operation amount of the master arm operated by the operator Op.

The slave control circuit 400 controls operation of the slave arm or the like, which is the operation target of the master arm, according to the calculated driving amount. The slave control circuit 400 inputs a driving signal to the corresponding slave arm, while controls a magnitude and polarity of the driving signal so that a driving amount of the slave arm, which is the operation target, becomes a target driving amount, in accordance with a detection signal input from a position detector of the power unit according to the operation of the corresponding slave arm.

The master arms 500a and 500b are configured to include a plurality of link mechanisms. A position detector such as an incremental encoder is provided in each link configuring the link mechanisms. By detecting the operation of each of the links by the position detector, the operation amounts of the master arms 500a and 500b are detected by the input-processing circuit 700.

The medical manipulator system 1 of Fig. 1 uses the two master arms 500a and 500b to operate four slave arms and the medical manipulator system can appropriately switch the slave arms that are an operation target of the master arms. The switching is performed, for example, by the operator Op operating the operation part 600. The switching is unnecessary when the number of master arms and the number of slave arms are the same, and the operation targets have one-to-one correspondence to the master arms.

The operation part 600 includes various types of operating members such as a switching button for switching the slave arm that is an operation target of the master arms 500a and 500b, a scaling change switch configured to change an operation ratio between the master and the slave, and a foot switch configured to emergency-stop the system. When any one of the operating members configuring the operation part 600 is operated by the operator Op, an operation signal according to the operation of the corresponding operating member is input from the operation part 600 to the input-processing circuit 700.

The input-processing circuit 700 analyzes an operation signal from the master arms 500a and 500b and an operation signal from the operation part 600, generates a control signal for controlling the medical manipulator system 1 according to a result of analyzing the operation signals, and inputs the control signal to the slave control circuit 400.

The image-processing circuit 800 performs various types of image processing for displaying an image signal input from the slave control circuit 400 and generates image data to be displayed on the operator display 900a and the assistant display 900b. The operator display 900a and the assistant display 900b are configured by, for example, a liquid crystal display, and display an image based on the image data generated by the image-processing circuit 800 according to an image signal acquired by an observation instrument.

In the above-mentioned medical manipulator system 1, when the operator Op operates the master arms 500a and 500b, slave arms corresponding thereto and the surgical tools 240a to 240d attached to the slave arms are operated corresponding to movements of the master arms 500a and 500b. Accordingly, a desired procedure can be performed on the patient P.

The reference numeral 300 represents a drape for separating a portion in which a sterilization process is performed (clean area) and a portion in which the sterilization process is not performed (unclean area) in the medical manipulator system 1 according to the present embodiment.

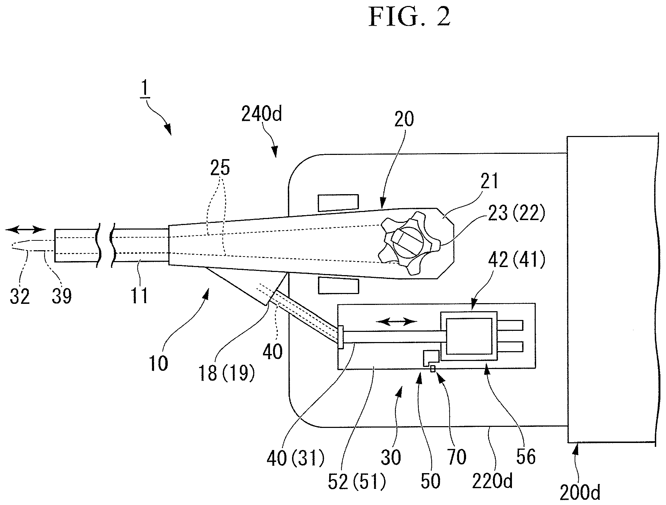

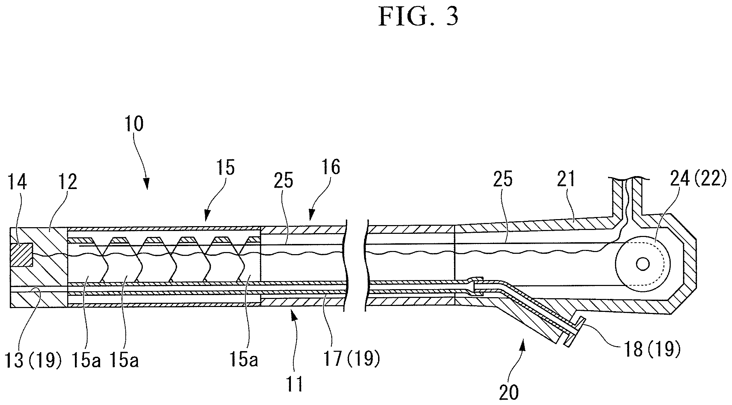

Next, a configuration of the surgical tool 240d of the medical manipulator system 1 according to the present embodiment will be described in detail. Fig. 2 is a schematic diagram showing a surgical tool of the medical manipulator system. Fig. 3 is a partial cross-sectional view showing a guide instrument of the surgical tool.

The surgical tool 240d includes a guide instrument 10 which is insertable into a body and a treatment tool 30 attached to the guide instrument 10. According to the present embodiment, both the guide instrument 10 and the treatment tool 30 are attached to the surgical power transmission adapter 220d. Both the guide instrument 10 and the treatment tool 30 can be attached to and detached from the surgical power transmission adapter 220d as necessary.

The guide instrument 10 shown in Figs. 2 and 3 is an instrument inserted into a body to guide the treatment tool 30 to a treatment target in the body. The guide instrument 10 of the present embodiment includes an elongated inserting part 11 inserted into the body and an operation part 20 disposed at a proximal end part of the inserting part 11.

The guide instrument 10 of the present embodiment is an endoscope capable of observing the inside of the body. It is not essential that the guide instrument is able to observe the inside of the body.

As shown in Fig. 3, the inserting part 11 includes a rigid distal end part 12, an imaging part 14, an active bending part 15, and a flexible tube part 16.

The rigid distal end part 12 includes a channel pipe 13 into which the treatment tool 30 is inserted. The imaging part 14 which will be described below is disposed in the rigid distal end part 12.

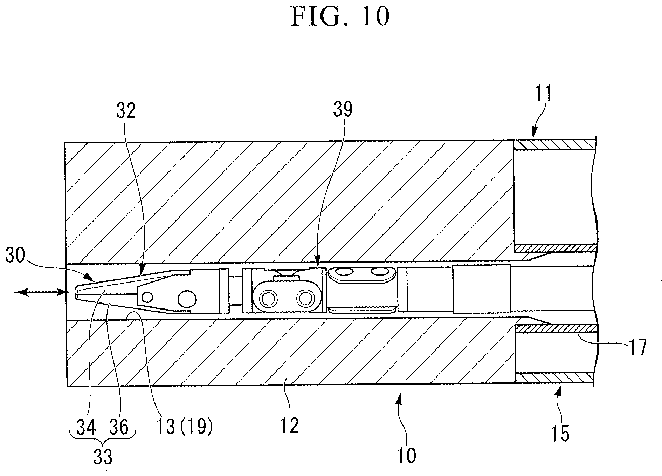

The channel pipe 13 is a pipe formed in a linear shape. A treatment part 32 and a joint part 39 which will be described below of the treatment tool 30 can be inserted into the channel pipe 13 (see Fig. 10). An inner diameter of the channel pipe 13 is set to keep the treatment part 32 and the joint part 39 substantially linear. A length of the channel pipe 13 along a central line of the channel pipe 13 is a length capable of accommodating the treatment part 32 and the joint part 39 of the treatment tool 30 in the channel pipe 13.

The imaging part 14 includes, for example, an optical system (not shown) and an image sensor, and can capture an image of an outside of the inserting part 11. The configuration of the imaging part 14 is not particularly limited.

The active bending part 15 is a tubular part disposed between the rigid distal end part 12 and the flexible tube part 16. The active bending part 15 includes, for example, a plurality of cylindrical joint rings 15a bendably connected along a longitudinal axis direction of the inserting part 11. In this case, an angle wire 25, which will be described below, is connected to a joint ring at a distal end side of the plurality of joint rings. The active bending part 15 can be towed by the angle wire 25 and deformed in a bent shape corresponding to an operation in the operation part 20.

The flexible tube part 16 is a flexible cylindrical member in which a signal line (not shown) connected to the imaging part 14 or the angle wire 25 which will be described below is disposed. The flexible tube part 16 includes a channel tube 17 having a distal end connected to a proximal end of the channel pipe 13 of the rigid distal end part 12, and a treatment tool introduction opening 18 connected to a proximal end of the channel tube 17.

The channel tube 17 is a tube into which an insertion body 31 of the treatment tool 30 is insertable. The channel tube 17 is flexibly deformable according to deformation of the flexible tube part 16. The distal end of the channel tube 17 is in communication with the proximal end of the channel pipe 13. The proximal end of the channel tube 17 is in communication with a distal end of the treatment tool introduction opening 18.

The treatment tool introduction opening 18 is disposed in the operation part 20. The treatment tool introduction opening 18 is formed of a rigid or flexible cylindrical member into which the insertion body 31 (see Fig. 2) of the treatment tool 30 is insertable. The treatment tool introduction opening 18 is disposed outside the body when the treatment tool 30 is in use.

In the present embodiment, as shown in Fig. 3, a treatment tool channel 19 into which the insertion body 31 of the treatment tool 30 is inserted is formed by the channel pipe 13 provided in the rigid distal end part 12 and the channel tube 17 and the treatment tool introduction opening 18 provided at the flexible tube part 16. In the present embodiment, other elements are not essential as long as the guide instrument 10 includes the treatment tool channel 19.

The operation part 20 includes a case body 21 disposed at a proximal end of the flexible tube part 16, and an angle knob 22 attached to the case body 21.

The angle knob 22 includes a rotatable dial part 23 disposed at an outer portion of the case body 21, and a sprocket or pulley (not shown) connected to the dial part 23 and disposed inside the case body 21 (hereinafter referred to as "sprocket or the like 24."). The dial part 23 can be operated using the master arms 500a and 500b shown in Fig. 1 by a driving device (not shown).

The angle wire 25 is connected to the sprocket or the like 24 of the angle knob 22. Accordingly, a bending operation of the active bending part 15 is possible by the angle wire 25 being operated through the sprocket or the like 24 by rotating the angle knob 22.

The angle wire 25 is connected to the active bending part 15 and the angle knob 22 and transmits power of a rotating operation of the dial part 23 of the angle knob 22 to the active bending part 15. In the present embodiment, a plurality of angle wires 25 are provided corresponding to a direction in which the active bending part 15 is bendable. The plurality of angle wires 25 can be independently operated by the angle knob 22.

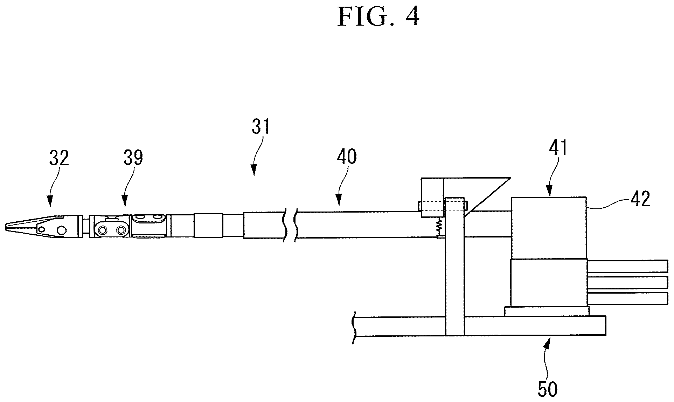

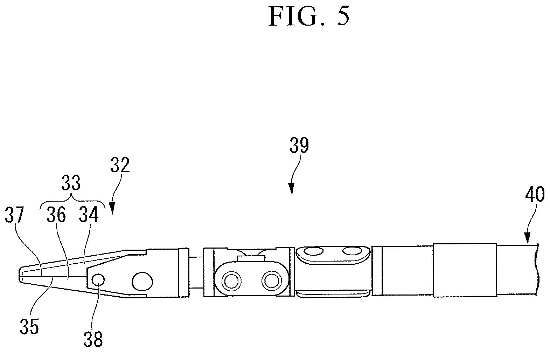

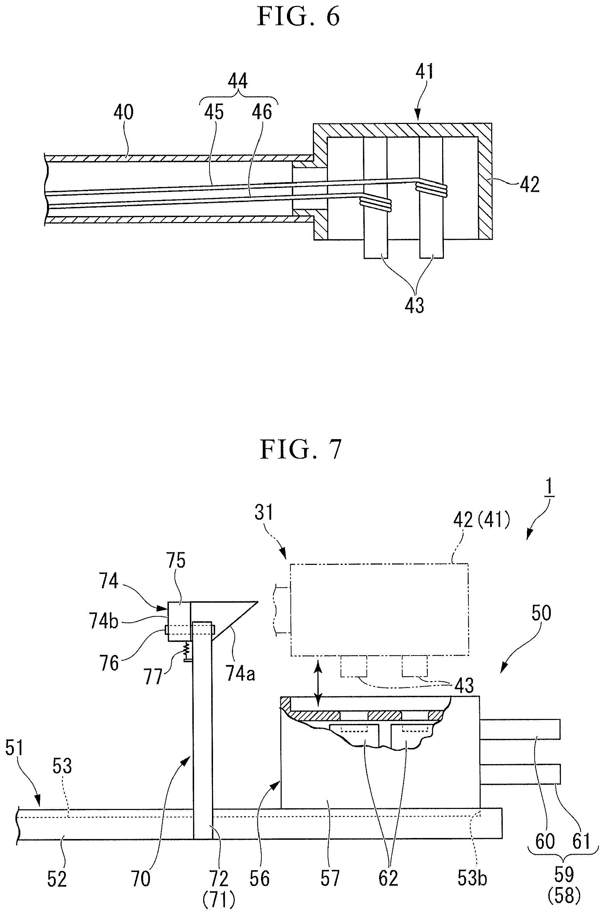

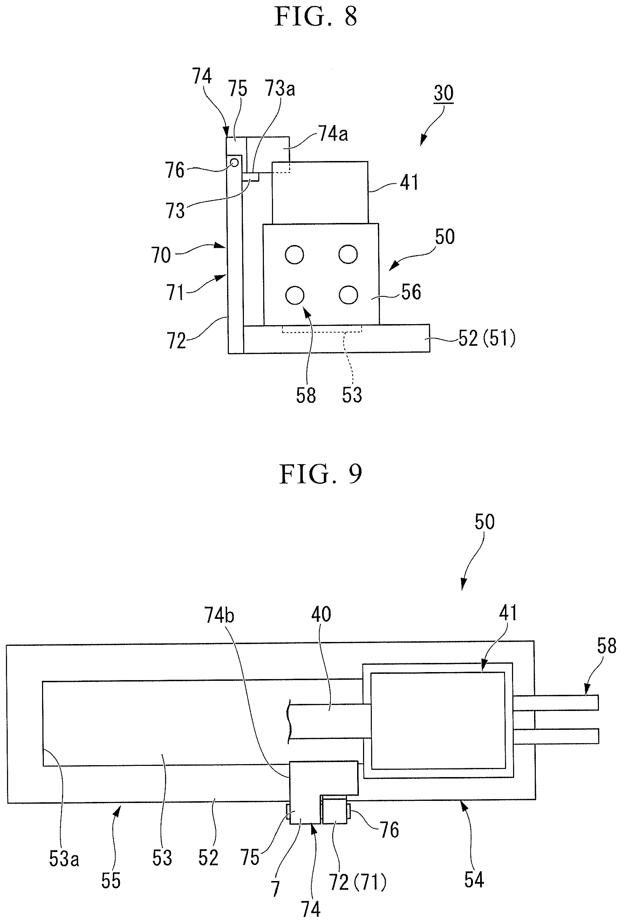

Next, a configuration of the treatment tool 30 used by being inserted into the treatment tool channel 19 of the guide instrument 10 of the present embodiment will be described. Fig. 4 is a schematic diagram showing a treatment tool of the surgical tool. Fig. 5 is an enlarged partial cross-sectional view showing a distal end part of an insertion body of the treatment tool. Fig. 6 is a partial cross-sectional view showing an attachable/detachable part of the insertion body. Fig. 7 is a side view showing a driver body of the treatment tool. Fig. 8 is a rear view of the driver body. Fig. 9 is a plan view of the driver body.

The treatment tool 30 shown in Figs. 2 and 4 includes the insertion body 31 which is inserted into the treatment tool channel 19 and is projectable from and retractable into a distal end of the inserting part 11, and a driver body 50 disposed at a proximal end of the insertion body 31.

As shown in Figs. 4 and 6, the insertion body 31 includes the treatment part 32, the joint part 39, a flexible part 40, an attachable/detachable part 41, and transmitting members 44.

To perform treatment on a treatment target in the body, the treatment part 32 shown in Figs. 4 and 5 is provided at the distal end of the insertion body 31. The configuration of the treatment part 32 is not particularly limited. Known configurations for performing grasping, dissection, cauterization, suturing, observation and other treatments on a treatment target may be appropriately selected and adopted as the treatment part 32. For example, examples of the treatment part 32 include a forceps, a knife, a marking device, a suture device, and the like. Hereinafter, a configuration capable of grasping a tissue and the like present at a treatment target will be described as an example of the treatment part 32.

As shown in Fig. 5, the treatment part 32 of the present embodiment includes a pair of jaws 33 and a rotation shaft member 38 configured to connect the pair of jaws 33 to each other.

The pair of jaws 33 include a first jaw 34 having a first grasping surface 35 for grasping a treatment target and a second jaw 36 having a second grasping surface 37 for grasping the treatment target. The first jaw 34 and the second jaw 36 can be opened and closed by rotating about a central line of the rotation shaft member 38 as a center of rotation. The first jaw 34 and the second jaw 36 are opened and closed by a force transmitted to the first jaw 34 and the second jaw 36 via a wire (a first transmitting member 45 which will be described below) from the driver body 50 which will be described below. Accordingly, the pair of jaws 33 can grasp tissue while the tissue is fitted between the first grasping surface 35 and the second grasping surface 37.

The joint part 39 is interposed between the treatment part 32 and the flexible part 40. The joint part 39 has one or more degrees of freedom. The joint part 39 is bent by a force transmitted to the joint part 39 via a wire (a second transmitting member 46 which will be described below) from the driver body 50 which will be described below. For example, the joint part 39 may swing the treatment part 32 by receiving a force from the second transmitting member 46 and being bent and deformed by rotating about a predetermined rotation shaft as a center of rotation. A plurality of predetermined rotation shafts of the joint part 39 that are spaced apart from each other may be provided. When the driver body 50 does not apply a force to the second transmitting member 46, the joint part 39 can be freely bent and deformed by an external force within a range of the degree of freedom thereof.

The flexible part 40 is a cylindrical member having flexibility. The transmitting members 44 such as the first transmitting member 45 for operating the treatment part 32 or the second transmitting member 46 for operating the joint part 39 are disposed inside the flexible part 40 of the present embodiment. The flexible part 40 is longer than an overall length of the treatment tool channel 19. Accordingly, when a distal end of the flexible part 40 is disposed at the distal end of the treatment tool channel 19, a proximal end of the flexible part 40 is disposed at an outer portion of the treatment tool introduction opening 18.

As shown in Figs. 4 and 6, the attachable/detachable part 41 is disposed at the proximal end of the flexible part 40. The attachable/detachable part 41 includes a frame body 42 and a rotator part 43.

The frame body 42 is fixed to the proximal end of the flexible part 40. The frame body 42 is a member which is attachable to and detachable from a substrate 57 provided at a power source unit 56 of the driver body 50 which will be described below. For example, a hook structure (not shown) which is engaged with the substrate 57 of the power source unit 56 is formed in the frame body 42. When the frame body 42 and the substrate 57 are engaged, the rotator part 43 is connected to a power-transmitting part 62 of the power source unit 56 by having a predetermined positional relationship therewith.

The rotator part 43 is disposed inside the frame body 42. The rotator part 43 includes a plurality of shaft elements (not shown) around which the first transmitting member 45 which is a wire configured to operate the treatment part 32 and the second transmitting member 46 which is a wire configured to operate the joint part 39 are wound. The first transmitting member 45 and the second transmitting member 46 are towed independently by the plurality of shaft elements configuring the rotator part 43 receiving power from a power source 58 of the power source unit 56 and rotating.

The transmitting members 44 include the first transmitting member 45 configured to open and close the pair of jaws 33 of the treatment part 32 and the second transmitting member 46 configured to curve the joint part 39. In the present embodiment, each of the first transmitting member 45 and the second transmitting member 46 is a wire.

As shown in Fig. 7, the driver body 50 includes a linear motion guide 51, the power source unit 56 disposed at the linear motion guide 51, and a regulating part 70 fixed to the linear motion guide 51.

As shown in Fig. 2, the linear motion guide 51 is attachable to the surgical power transmission adapter 220d of the slave arm 200d. The linear motion guide 51 includes a base member 52 which is fixable to the surgical power transmission adapter 220d and a guide rail 53 provided at the base member 52.

The base member 52 and the guide rail 53 are fixed to the surgical power transmission adapter 220d in a state in which a positional relationship with the guide instrument 10 is defined so that the guide rail 53 extends substantially toward the treatment tool introduction opening 18 of the guide instrument 10. The power source unit 56 which will be described below is advanceably and retractably connected to the guide rail 53. Accordingly, the guide rail 53 movably supports the power source unit 56 in one direction so that the power source unit 56 moves toward or away from the treatment tool introduction opening 18. In the present embodiment, a direction toward the treatment tool introduction opening 18 in an extending direction of the guide rail 53 is referred to as a direction facing a distal end 53a of the guide rail 53. In the present embodiment, a direction away from the treatment tool introduction opening 18 in the extending direction of the guide rail 53 is referred to as a direction facing a proximal end 53b of the guide rail 53.

As shown in Figs. 7 and 9, the guide rail 53 includes a first region 54 which is closer to the proximal end than the regulating part 70 and a second region 55 which is closer to the distal end than the regulating part 70, with a position of the regulating part 70 as a boundary between the first region 54 and the second region 55. In the extending direction of the guide rail 53, when the power source unit 56 is disposed in the first region 54 which is closer to the proximal end than the regulating part 70, the treatment part 32 of the treatment tool 30 is disposed in the channel pipe 13 (see Fig. 10). In the extending direction of the guide rail 53, when the power source unit 56 is disposed in the second region 55 at the distal end side over the regulating part 70 from the first region 54, the treatment part 32 of the treatment tool 30 and the joint part 39 are at positions protruding from the distal end of the channel pipe 13 according to a position of the power source unit 56 on the guide rail 53.

As shown in Fig. 7, the power source unit 56 includes the substrate 57, the power source 58, and the power-transmitting part 62.

The substrate 57 is connected to the guide rail 53 and supported by the guide rail 53. The substrate 57 is movable along the guide rail 53 and can be connected to the frame body 42 by being engaged with the above-described frame body 42 disposed at the proximal end of the insertion body 31. The substrate 57 is attachable to and detachable from the frame body 42. Accordingly, the power source unit 56 is attachable to and detachable from the insertion body 31.

The power source 58 is attached to the substrate 57. The power source 58 has a plurality of motors 59 which rotate by receiving supply of power. The plurality of motors 59 are provided to correspond to the transmitting members 44 provided at the insertion body 31. As shown in Figs. 6 and 7, for example, the plurality of motors 59 include a first motor 60 configured to open and close the pair of jaws 33 of the treatment part 32 by towing the first transmitting member 45 and a second motor 61 configured to curve the joint part 39 by towing the second transmitting member 46. The power source 58 of the present embodiment is operable using the master arms 500a and 500b shown in Fig. 1. In a case in which the treatment part 32 has a configuration that does not require a mechanical operation, the first motor 60 may not be provided.

As shown in Fig. 7, the power-transmitting part 62 is attached to the substrate 57. The power-transmitting part 62 is operated by power generated by the power source 58 to transmit the power generated by the power source 58 to the rotator part 43 of the attachable/detachable part 41 of the insertion body 31. The power-transmitting part 62 according to the present embodiment includes a plurality of transmitting elements (not shown) disposed in the substrate 57 to be simultaneously attached to or detached from each of the shaft elements of the attachable/detachable part 41. Accordingly, the attachable/detachable part 41 is connected to the power-transmitting part 62 by the frame body 42 being engaged with the substrate 57, and a driving force can be transmitted from the power source 58 to the treatment part 32 and the joint part 39 of the insertion body 31.

As shown in Figs. 7, 8, and 9, the regulating part 70 includes a support body 71 fixed to the base member 52 of the linear motion guide 51, a rotator 74 (an opening/closing member) connected to the support body 71 via a shaft member 76, and a biasing member 77 connected to the support body 71 and the rotator 74.

The support body 71 includes a column member 72 disposed at a position which does not interfere with movement of the power source unit 56 and the attachable/detachable part 41 along the guide rail 53, and a placing table part 73 disposed at an end part of the column member 72.

A through-hole extending in parallel with the extending direction of the guide rail 53 is formed in the column member 72. The above-mentioned shaft member 76 is inserted into the through-hole formed in the column member 72.

The placing table part 73 includes a placing surface 73a extending in a direction crossing a central line of the column member 72 (in the present embodiment, a direction orthogonal to the central line of the column member 72). The rotator 74 is placed on the placing surface 73a of the placing table part 73. As shown in Figs. 7 and 8, in a state in which the rotator 74 is in contact with the placing surface 73a, a clearance through which the power source unit 56 may pass is generated between the guide rail 53 and the rotator 74. In a state in which the rotator 74 is in contact with the placing surface 73a, the frame body 42 of the attachable/detachable part 41 may come into contact with the rotator 74 in a state in which the attachable/detachable part 41 is attached to the power source unit 56 (see Fig. 8). The placing surface 73a of the placing table part 73 supports the rotator 74 so that the rotator 74 is disposed on a movement path of the frame body 42.

As shown in Fig. 7, the rotator 74 includes a connecting part 75 having a through-hole extending in parallel with the extending direction of the guide rail 53, a tapered surface 74a (a first surface) oriented toward the direction facing the proximal end 53b of the guide rail 53 and toward the guide rail 53, and an abutting surface 74b (a second surface) oriented toward the direction facing the distal end 53a of the guide rail 53. The rotator 74 is supported by the support body 71 to be interposed in the boundary between the first region 54 and the second region 55.

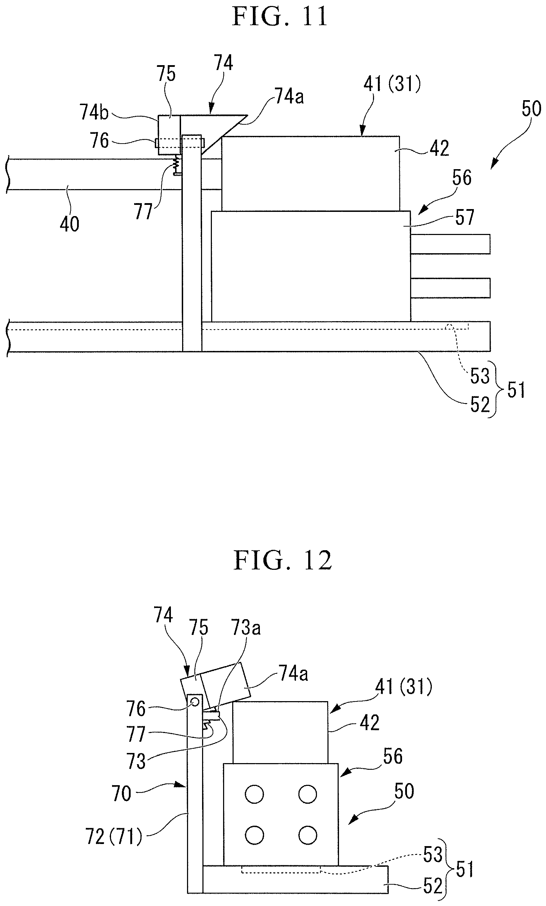

The connecting part 75 includes the through-hole into which the shaft member 76 configured to connect the rotator 74 and the column member 72 is inserted and makes the rotator 74 rotatable with a central line of the through-hole as a center of rotation with respect to the column member 72 (see Fig. 12). The rotator 74 is rotatable with respect to the column member 72 about a rotation axis extending along the extending direction of the guide rail 53. In a state in which the rotator 74 is rotated away from the placing surface 73a of the placing table part 73, the power source unit 56 which is engaged with the attachable/detachable part 41 may pass through the regulating part 70 and advance and retract along the guide rail 53 together with the attachable/detachable part 41.

As shown in Fig. 8, the tapered surface 74a is a surface having a clearance through which the power source unit 56 can pass formed between itself and the guide rail 53 and disposed at a position spaced apart from the guide rail 53 to an extent that the tapered surface 74a can come into contact with the frame body 42 of the attachable/detachable part 41. In a state in which the attachable/detachable part 41 of the insertion body 31 is engaged with the power source unit 56 of the driver body 50, when the power source unit 56 moves along the guide rail 53 from the proximal end 53b of the guide rail 53 toward the distal end 53a of the guide rail 53, the frame body 42 of the attachable/detachable part 41 of the insertion body 31 comes into contact with the tapered surface 74a (see Fig. 11). The rotator 74 moves away from the placing surface 73a by the frame body 42 pushing the tapered surface 74a. In the present embodiment, the rotator 74 moves away from the placing surface 73a by being pushed by the frame body 42 and rotated about the central line of the through-hole of the connecting part 75 as the center of rotation and returns to a state in which the rotator 74 is in contact with the placing surface 73a when the frame body 42 is spaced apart from the rotator 74.

The abutting surface 74b is a surface orthogonal to the extending direction of the guide rail 53. The abutting surface 74b has a clearance through which the power source unit 56 can pass formed between itself and the guide rail 53 and disposed at a position spaced apart from the guide rail 53 to an extent that the abutting surface 74b can come into contact with the frame body 42 of the attachable/detachable part 41. In the state in which the attachable/detachable part 41 of the insertion body 31 is engaged with the power source unit 56 of the driver body 50, when the power source unit 56 moves along the guide rail 53 from the distal end 53a of the guide rail 53 toward the proximal end 53b of the guide rail 53, the attachable/detachable part 41 of the insertion body 31 comes into contact with the abutting surface 74b. In a state in which the attachable/detachable part 41 of the insertion body 31 is in contact with the abutting surface 74b, the attachable/detachable part 41 moving toward the proximal end 53b of the guide rail 53 is regulated by the rotator 74.

Instead of the abutting surface 74b, an abutting part having a shape for applying a force for pushing the attachable/detachable part 41 back toward the distal end 53a of the guide rail 53 when the attachable/detachable part 41 is moved toward the proximal end 53b of the guide rail 53 may be formed at the rotator 74.

The biasing member 77 shown in Fig. 7 is a member configured to bias the rotator 74 in a direction in which the rotator 74 moves toward the placing surface 73a of the placing table part 73. In the present embodiment, the biasing member 77 is formed of a tension coil spring configured to connect the column member 72 and the rotator 74. In a case in which the rotator 74 is manually moved so that the rotator 74 moves away from the placing surface 73a, when the rotator 74 is released from the hand, the rotator 74 moves in reverse due to an action of the biasing member 77 until the rotator 74 comes into contact with the placing surface 73a.

In the present embodiment, when the rotator 74 is moved away from the placing surface 73a by the frame body 42 pushing the tapered surface 74a (see Fig. 12) and the frame body 42 is spaced apart from the rotator 74, the rotator 74 moves in reverse so that the rotator 74 moves toward the placing surface 73a until the rotator 74 comes into contact with the placing surface 73a.

A position of the regulating part 70 in the driver body 50 is set to correspond to a configuration of the treatment tool 30. In the present embodiment, the position of the regulating part 70 in the driver body 50 is determined so that the joint part 39 is exposed from the distal end of the treatment tool channel 19 when the attachable/detachable part 41 is in contact with the abutting surface 74b.

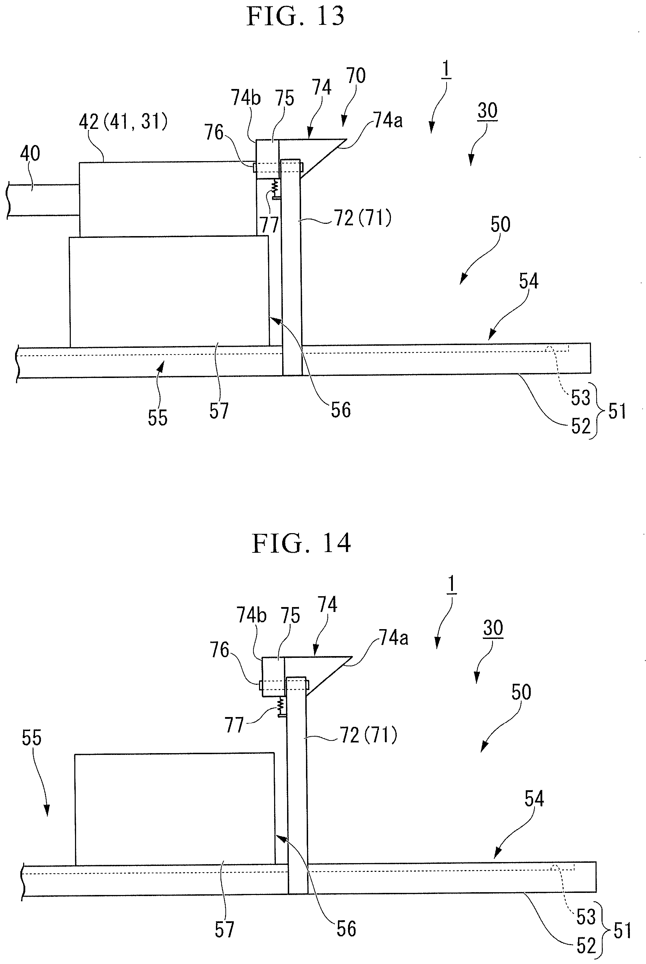

An operation of the medical manipulator system 1 according to the present embodiment will be described. Fig. 10 is a partial cross-sectional view showing a process in which the insertion body is inserted into a treatment tool channel of the guide instrument. Fig. 11 is a side view showing a process in which the attachable/detachable part of the insertion body moves along a guide rail of the driver body. Fig. 12 is a rear view showing a process in which the attachable/detachable part of the insertion body moves along the guide rail of the driver body. Fig. 13 is a side view showing a process in which the attachable/detachable part of the insertion body moves along the guide rail of the driver body. Fig. 14 is a side view showing a process in which, after the insertion body is detached from a power source unit of the driver body, the power source unit moves.

When the medical manipulator system 1 according to the present embodiment shown in Fig. 1 is in use, the surgical tools 240a to 240d can be attached to the slave arms 200a to 200d as necessary.

For example, when the surgical tool 240d is used by being attached to the slave arm 200d as shown in Fig. 2, the guide instrument 10 and the treatment tool 30 are attached in advance to the surgical power transmission adapter 220d of the slave arm 200d. First, the inserting part 11 of the guide instrument 10 is inserted into the body as shown in Fig. 1, and the guide instrument 10 is attached to the surgical power transmission adapter 220d as shown in Fig. 2. The distal end of the inserting part 11 is guided to a treatment target while observation is performed using the imaging part 14 as needed. By operating the angle knob 22 and bending and deforming the active bending part 15, the inserting part 11 can be moved along a bent portion in the body or the inserting part 11 can be moved to avoid an obstacle.

When the distal end of the inserting part 11 is guided to a position near the treatment target, the insertion body 31 of the treatment tool 30 is inserted into the treatment tool channel 19 through the treatment tool introduction opening 18 of the inserting part 11. The attachable/detachable part 41 is detached from the driver body 50. In the state in which the attachable/detachable part 41 is detached from the driver body 50, the treatment part 32 and the joint part 39 are freely movable by receiving an external force. As a result, the treatment tool 30 and the joint part 39 pass through an inner portion of the treatment tool channel 19 and are smoothly guided toward the distal end along the treatment tool channel 19.

When the insertion body 31 is inserted into the treatment tool channel 19, an operator of the medical manipulator according to the present embodiment connects the power source unit 56 to the attachable/detachable part 41. As shown in Fig. 7, the frame body 42 is fitted to the substrate 57 so that the frame body 42 of the attachable/detachable part 41 is engaged with the substrate 57 of the power source unit 56. When engaging the substrate 57 with the frame body 42, first, the substrate 57 is moved along the guide rail 53 so that the substrate 57 is disposed at the most proximal end of the guide rail 53 to engage the frame body 42 with the substrate 57 at the proximal end 53b of the guide rail 53. The treatment part 32 and the joint part 39 of the insertion body 31 are accommodated in the treatment tool channel 19.

As shown in Fig. 7, when the frame body 42 is engaged with the substrate 57, the rotator part 43 and the power-transmitting part 62 are connected. When the substrate 57 is at the proximal end 53b of the guide rail 53, the treatment part 32 and the joint part 39 of the treatment tool 30 is disposed in the channel pipe 13 formed in the rigid distal end part 12 of the inserting part 11 of the guide instrument 10 as shown in Fig. 10. Consequently, the treatment part 32 and the joint part 39 are linear along the channel pipe 13.

After the frame body 42 is engaged with the substrate 57, when an input which indicates that the frame body 42 and the substrate 57 are engaged is transmitted to the slave control circuit 400 by, for example, a manual input by the operator, the slave control circuit 400 stores a state of the power source 58 in a state corresponding to the joint part 39 which is linear in the channel pipe 13 as an initial state. To store the initial state, a predetermined calibration operation may be performed in the channel pipe 13 with respect to the treatment part 32 and the joint part 39.

After the initial state is stored, each power source 58 is controlled to operate with the initial state as an original position. With respect to the pair of jaws 33 (see Fig. 10), a state of the power source 58 corresponding to a state in which the first jaw 34 and the second jaw 36 are in contact and stopped when the first jaw 34 and the second jaw 36 are moved in a closing direction of the pair of jaws 33 is stored in the slave control circuit 400 as an initial position.

When setting an initial state in the channel pipe 13 is finished, treatment using the treatment tool 30 can be performed on the treatment target. As shown in Fig. 11, the operator grasps the power source unit 56 or the attachable/detachable part 41 and integrally moves the power source unit 56 and the attachable/detachable part 41 from the proximal end side toward the distal end side along the guide rail 53 of the linear motion guide 51. In the process in which the power source unit 56 and the attachable/detachable part 41 move toward the distal end side along the guide rail 53, the frame body 42 of the attachable/detachable part 41 comes into contact with the tapered surface 74a of the rotator 74. When the frame body 42 moves further toward the distal end 53a of the guide rail 53, the frame body 42 reaches a state in which the frame body 42 pushes the tapered surface 74a as shown in Fig. 12. The rotator 74 rotates against a biasing force of the biasing member 77 about the central line of the through-hole of the connecting part 75 as the center of rotation to move away from the guide rail 53. Accordingly, the power source unit 56 and the attachable/detachable part 41 can integrally move toward the distal end 53a of the guide rail 53 over the regulating part 70.

When the power source unit 56 and the attachable/detachable part 41 move toward the distal end side over the regulating part 70 and the frame body 42 is spaced apart from the rotator 74, the biasing member 77 moves the rotator 74 so that the rotator 74 returns to the original position at which the rotator 74 is in contact with the placing surface 73a of the placing table part 73. Accordingly, as shown in Fig. 13, the abutting surface 74b of the rotator 74 is disposed at a position at which the abutting surface 74b is capable of regulating movement of the frame body 42 along the guide rail 53. When the power source unit 56 and the attachable/detachable part 41 moves further toward the distal end past the regulating part 70, a state in which the power source unit 56 and the attachable/detachable part 41 cannot move further toward the proximal end past the regulating part 70 is established automatically by the biasing member 77 in the state in which the power source unit 56 and the attachable/detachable part 41 are engaged with each other.

In a state in which the power source unit 56 and the attachable/detachable part 41 are at the distal end side over the regulating part 70, because the treatment part 32 and the joint part 39 of the treatment tool 30 (see Fig. 4) protrude from the distal end of the treatment tool channel 19, a desired operation can be performed with respect to the treatment part 32 and the joint part 39 by using each of the power sources 58 of the power source unit 56. The master arms 500a and 500b shown in Fig. 1 are used in operations of the treatment part 32 and the joint part 39.

When use of the treatment tool 30 is finished, the treatment tool 30 can be separated from the guide instrument 10 shown in Fig. 2. When the joint part 39 of the treatment tool 30 is bent, the joint part 39 is caught in the distal end of the channel pipe 13. In the present embodiment, when the treatment tool 30 is separated from the guide instrument 10, the rotator part 43 is spaced apart from the power-transmitting part 62 by separating the attachable/detachable part 41 from the power source unit 56 (see Figs. 13 and 14). The frame body 42 is withdrawn from the substrate 57 by releasing the engagement between the substrate 57 of the power source unit 56 and the frame body 42 of the attachable/detachable part 41. Accordingly, because the treatment part 32 and the joint part 39 reach the state in which the treatment part 32 and the joint part 39 are freely movable by the external force, the treatment part 32 and the joint part 39 are smoothly withdrawn by being deformed along the treatment tool channel 19 by the operator withdrawing the insertion body 31 from the treatment tool channel 19.

As described above, in the medical manipulator system 1 according to the present embodiment, in the state in which the power source unit 56 and the attachable/detachable part 41 are connected, the power source unit 56 and the attachable/detachable part 41 cannot move from the distal end side of the regulating part 70 toward the proximal end side thereof over the regulating part 70. Consequently, the connection between the power source unit 56 and the attachable/detachable part 41 needs to be released to withdraw the insertion body 31 from the treatment tool channel 19. In the medical manipulator system 1 according to the present embodiment, the regulating part 70 regulates the insertion body 31 so that the insertion body 31 cannot be drawn into the treatment tool channel 19 while a force that bends the joint part 39 is applied to the joint part 39. As a result, according to the medical manipulator system 1 of the present embodiment, an erroneous operation in which the insertion body 31 is drawn into the treatment tool channel 19 while a force that bends the joint part 39 is applied cannot occur.

In the present embodiment, when the attachable/detachable part 41 is detached from the power source unit 56, the power source unit 56 can be moved toward the proximal end 53b of the guide rail 53 over the regulating part 70. When the attachable/detachable part 41 is not attached to the power source unit 56, the power source unit 56 is movable along the guide rail 53. As a result, the work of replacing the insertion body 31 of the treatment part 32 is easy.

### (Modified example 1-1)

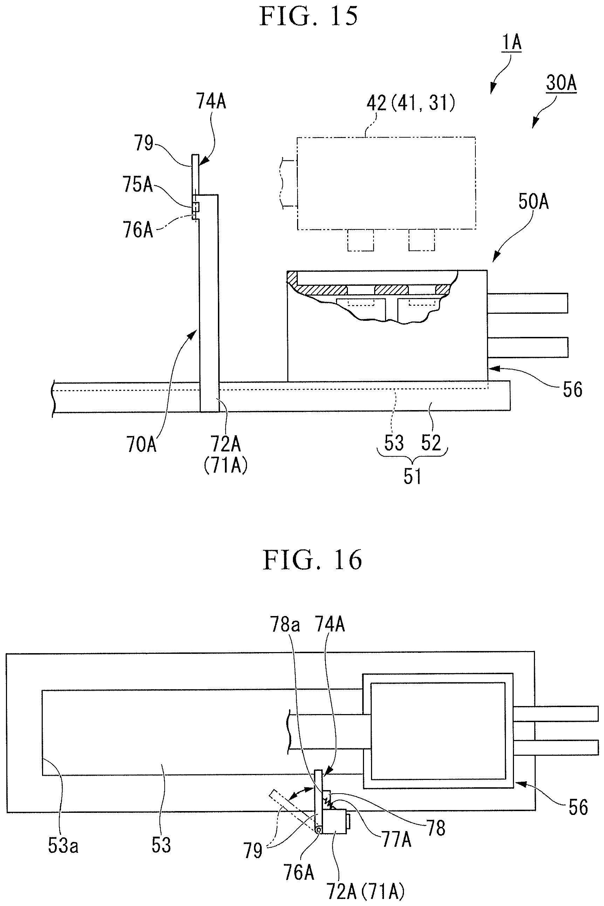

A modified example of the embodiment will be described. Fig. 15 is a side view showing a configuration of the present modified example. Fig. 16 is a plan view showing the configuration of the present modified example.

As shown in Figs. 15 and 16, a medical manipulator system 1A of the present modified example includes a treatment tool 30A having a configuration different from that of the treatment tool 30 disclosed in the first embodiment, instead of the treatment tool 30 disclosed in the first embodiment.

The treatment tool 30A of the present modified example includes the insertion body 31 disclosed in the first embodiment and a driver body 50A provided instead of the driver body 50 disclosed in the first embodiment.

The driver body 50A includes the linear motion guide 51 and the power source unit 56 disclosed in the first embodiment and a regulating part 70A provided instead of the regulating part 70 disclosed in the first embodiment.

The regulating part 70A includes a support body 71A, a rotator 74A (an opening/closing member), a shaft member 76A, and a biasing member 77A.

The support body 71A includes a column member 72A and a regulating plate 78.

Like the column member 72 disclosed in the first embodiment, the column member 72A is provided at the base member 52. A through-hole extending in a direction orthogonal to the extending direction of the guide rail 53 and having the shaft member 76A disposed therein is formed in the column member 72A.

The regulating plate 78 is a plate-shaped member having a contact surface 78a oriented toward the distal end 53a of the guide rail 53 and with which the rotator 74A can come into contact. The regulating plate 78 is fixed to the column member 72A. The contact surface 78a of the regulating plate 78 has the same action as the placing surface 73a of the placing table part 73 disclosed in the first embodiment in such a way that the contact surface 78a supports the rotator 74A so that the rotator 74A is disposed on the movement path of the frame body 42.

The rotator 74A (the opening/closing member) includes a connecting part 75A and a plate member 79.

The connecting part 75A is a part in which a through-hole formed coaxially with the through-hole of the column member 72A and having the shaft member 76A disposed therein is formed. The connecting part 75A connects the column member 72A and the rotator 74A by the shaft member 76A so that the rotator 74A is rotatable about a central line of the through-hole of the connecting part 75A as the center of rotation with respect to the column member 72A. The rotator 74A is rotatable about a rotation axis extending in a direction orthogonal to the extending direction of the guide rail 53 with respect to the column member 72A.

As the same with the rotator 74 disclosed in the first embodiment, the plate member 79 is a member that is insertable into and removable from the movement path of the frame body 42, and is formed in a flat plate shape in the present embodiment. In the plate member 79, a surface oriented toward the first region 54 corresponds to the first surface in the above embodiment and a surface oriented toward the second region 55 corresponds to the second surface in the above embodiment.

The biasing member 77A biases the rotator 74A with respect to the column member 72A in a direction in which the plate member 79 comes into contact with the contact surface 78a.

In the present modified example, when the power source unit 56 and the attachable/detachable part 41 integrally moves from the proximal end 53b of the guide rail 53 toward the distal end while the attachable/detachable part 41 is connected to the power source unit 56, the frame body 42 of the attachable/detachable part 41 comes into contact with the plate member 79. With the central line of the through-hole of the connecting part 75A provided at the rotator 74A as the center of rotation, the frame body 42 rotates the plate member 79 while pushing the plate member 79 toward the distal end 53a of the guide rail 53. A space for allowing the power source unit 56 and the attachable/detachable part 41 to integrally enter the second region 55 can be generated by pushing the rotator 74A out of the movement path of the frame body 42 against a biasing force of the biasing member 77A.

When the power source unit 56 and the attachable/detachable part 41 enter the second region 55 and the frame body 42 is spaced apart from the rotator 74A, the plate member 79 is moved by the biasing member 77A to a position at which the plate member 79 comes into contact with the contact surface 78a of the regulating plate 78 such that the rotator 74A is returned to the original position.

The plate member 79 cannot move toward the proximal end 53b of the guide rail 53 in the state in which the plate member 79 is in contact with the contact surface 78a of the regulating plate 78. Accordingly, when the power source unit 56 and the attachable/detachable part 41 disposed in the second region 55 are integrally moved toward the proximal end 53b of the guide rail 53, the attachable/detachable part 41 cannot move further toward the proximal end side from a state in which the attachable/detachable part 41 is in contact with the plate member 79. When the attachable/detachable part 41 is detached from the power source unit 56 in the second region 55, the power source unit 56 is movable to the first region 54 through a space between the plate member 79 and the guide rail 53 as in the first embodiment.

The configuration of the present modified example has the same effect as the above embodiment.

In the present modified example, a structure is simple as compared with the rotator 74 disclosed in the first embodiment, and the regulating part 70A of the present modified example can be more easily produced than the regulating part 70 of the first embodiment.

### (Modified example 1-2)

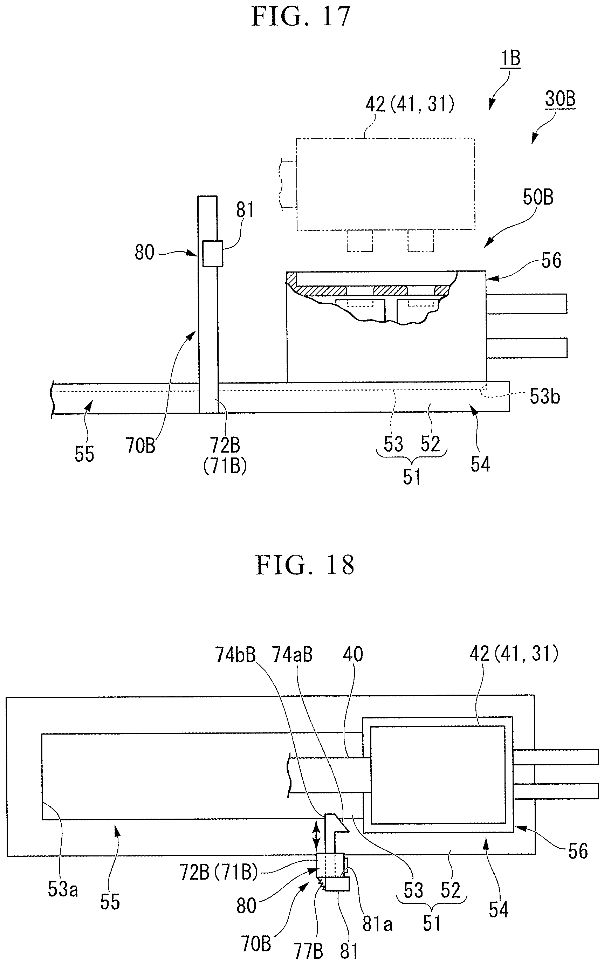

Another modified example of the first embodiment will be described. Fig. 17 is a side view showing a configuration of the present modified example. Fig. 18 is a plan view showing the configuration of the modified example.

As shown in Figs. 17 and 18, a medical manipulator system 1B according to the present modified example includes a treatment tool 30B having a configuration different from that of the treatment tool 30 disclosed in the first embodiment, instead of the treatment tool 30 disclosed in the first embodiment.

The treatment tool 30B of the present modified example includes the insertion body 31 disclosed in the first embodiment and a driver body 50B provided instead of the driver body 50 disclosed in the first embodiment.

The driver body 50B includes the linear motion guide 51 and the power source unit 56 disclosed in the first embodiment and a regulating part 70B provided instead of the regulating part 70 disclosed in the first embodiment.

The regulating part 70B includes a support body 71B, a movable body 81 (an opening/closing member), and a biasing member 77B.

The support body 71B includes a column member 72B having a holding part 80 configured to hold the movable body 81.

As the same with the first embodiment, the column member 72B is fixed to the base member 52. A through-hole orthogonal to the extending direction of the guide rail 53 and having a central line extending in a direction crossing the movement path of the frame body 42 is formed in the holding part 80 provided in the column member 72B.

The movable body 81 is a rod-shaped member advanceably and retractably inserted into the through-hole formed in the holding part 80. A tapered surface 74aB (a first surface) and an abutting surface 74bB (a second surface) which are the same as the tapered surface 74a and the abutting surface 74b disclosed in the first embodiment are formed in the vicinity of one end of both axial ends of the movable body 81, and a contact surface 81a is formed in the vicinity of the other end of the both axial ends of the movable body 81.

The tapered surface 74aB is a surface that is tilted with respect to the extending direction of the guide rail 53 and is oriented toward the proximal end 53b of the guide rail 53 and toward a side away from the column member 72B.

The abutting surface 74bB is a surface oriented toward the distal end side in the extending direction of the guide rail 53 and orthogonal to the extending direction of the guide rail 53.

The contact surface 81a is a surface formed toward the column member 72B to be in contact with the column member 72B when the tapered surface 74aB and the abutting surface 74bB are disposed on the movement path of the frame body 42.

The biasing member 77B biases the movable body 81 with respect to the column member 72B so that the tapered surface 74aB and the abutting surface 74bB are disposed on the movement path of the frame body 42.

In the present modified example, in a state in which no external force is applied to the movable body 81 other than from the biasing member 77B, the contact surface 81a is in contact with the column member 72B due to the biasing force of the biasing member 77B. The tapered surface 74aB and the abutting surface 74bB of the movable body 81 are disposed on the movement path of the frame body 42.

In a process in which the power source unit 56 and the attachable/detachable part 41 integrally move from the proximal end 53b of the guide rail 53 toward the distal end 53a of the guide rail 53, first, the frame body 42 of the attachable/detachable part 41 comes into contact with the tapered surface 74aB. When the power source unit 56 and the attachable/detachable part 41 integrally move further toward the distal end 53a of the guide rail 53, the frame body 42 pushes the movable body 81 against the biasing force of the biasing member 77B.

When the frame body 42 of the attachable/detachable part 41 enters the second region 55 and the frame body 42 is spaced apart from the movable body 81, the tapered surface 74aB and the abutting surface 74bB of the movable body 81 are disposed on the movement path of the frame body 42 again by the biasing force of the biasing member 77B.

In the state in which the attachable/detachable part 41 is attached to the power source unit 56, the frame body 42 abuts the abutting surface 74bB even when the power source unit 56 and the attachable/detachable part 41 attempt to integrally move from the second region 55 to the first region 54. Accordingly, the movable body 81 is not pushed out of the movement path of the frame body 42. As a result, movement of the frame body 42 is regulated. When the attachable/detachable part 41 is detached from the power source unit 56 in the second region 55, the power source unit 56 is movable to the first region 54 by passing through a portion between the movable body 81 and the guide rail 53.

The configuration of the present modified example has the same effect as the treatment tool 30 of the above embodiment.

### (Second embodiment)

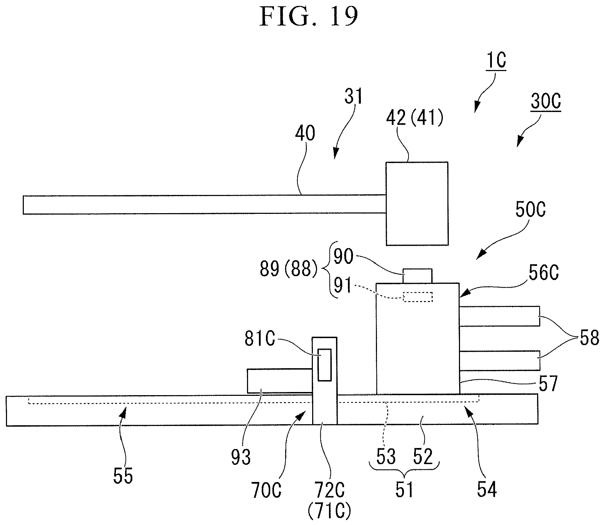

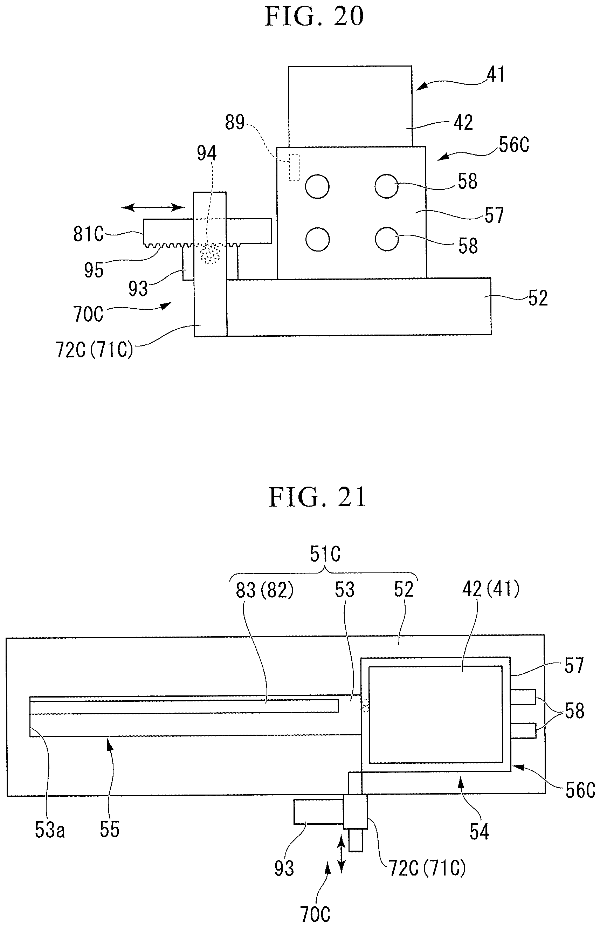

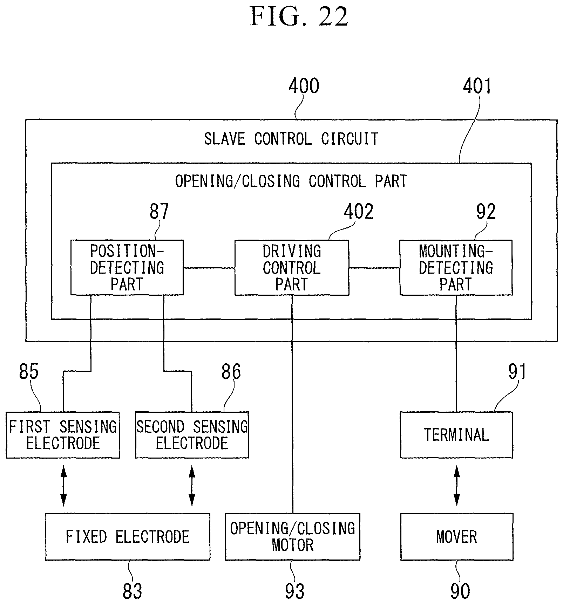

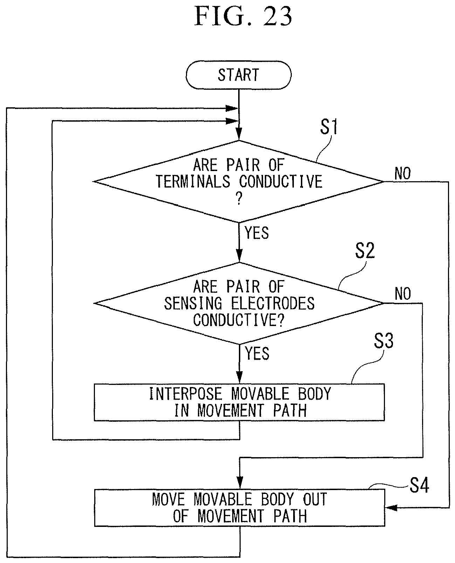

A second embodiment of the present invention will be described. In each embodiment which will be described below, like reference numerals from the first embodiment will be assigned to the same elements as in the above-described first embodiment, and same description thereof will be omitted. Fig. 19 is a side view showing a treatment tool in a medical manipulator system according to the present embodiment. Fig. 20 is a rear view of the treatment tool. Fig. 21 is a plan view of the treatment tool. Fig. 22 is a block diagram showing a partial configuration of a slave control circuit of the medical manipulator system according to the embodiment. Fig. 23 is a flowchart showing a part of processing in the slave control circuit.

As shown in Fig. 19, a medical manipulator system 1C according to the present embodiment includes a treatment tool 30C with a different configuration to the treatment tool 30 disclosed in the first embodiment, and instead of the treatment tool 30. The medical manipulator system 1C according to the present embodiment is different from the first embodiment in that the slave control circuit 400 disclosed in the first embodiment (see Fig. 1) can change a regulating state of a regulating part 70C (see Fig. 19) according to a position of a power source unit 56C disposed on the guide rail 53 (see Fig. 19).

The treatment tool 30C of the present embodiment includes the insertion body 31 disclosed in the first embodiment and a driver body 50C provided instead of the driver body 50 disclosed in the first embodiment.

The driver body 50C includes a linear motion guide 51C, the power source unit 56C, and the regulating part 70C.

As shown in Figs. 19 and 21, the linear motion guide 51C includes the base member 52 and the guide rail 53 disclosed in the first embodiment and a fixed electrode 83 disposed in the vicinity of the guide rail 53.

The fixed electrode 83 is an element of a position sensor 82 which will be described below. The fixed electrode 83 is formed of a conductor fixed to the base member 52. The fixed electrode 83 is provided to extend in the extending direction of the guide rail 53 in the second region 55 of the guide rail 53.

The power source unit 56C shown in Fig. 19 includes the substrate 57, the power source 58, and the power-transmitting part 62 (see Fig. 7) disclosed in the first embodiment, a pair of sensing electrodes 84 attached to the substrate 57, and a switch 89 attached to the substrate 57.

The pair of sensing electrodes 84 shown in Figs. 21, 24, and 25 are elements of the position sensor 82 which will be described below. The pair of sensing electrodes 84 include a first sensing electrode 85 and a second sensing electrode 86 which can come into contact with the fixed electrode 83 in the second region 55. When both of the first sensing electrode 85 and the second sensing electrode 86 are in contact with the fixed electrode 83, the first sensing electrode 85 and the second sensing electrode 86 are brought into a conductive state. When at least one of the first sensing electrode 85 and the second sensing electrode 86 is not in contact with the fixed electrode 83, the first sensing electrode 85 and the second sensing electrode 86 are brought into an insulating state. Each of the first sensing electrode 85 and the second sensing electrode 86 which constitute the pair of sensing electrodes 84 is electrically connected to a position-detecting part 87 of the slave control circuit 400.

The switch 89 shown in Fig. 19 is an element of a mounting sensor 88 which will be described below. The switch 89 includes a moving element 90 press-fitted by the frame body 42 when the frame body 42 of the insertion body 31 is attached to the substrate 57, and a pair of terminals 91 (e.g., microswitches) switched between the conductive state and the insulating state corresponding to a position of the moving element 90.

The moving element 90 provided in the switch 89 is press-fitted by the frame body 42 being engaged with the substrate 57 (see Figs. 19 and 20). The moving element 90 brings the pair of terminals 91 into the conductive state when the frame body 42 is engaged with the substrate 57.

As shown in Fig. 22, the pair of terminals 91 provided in the switch 89 are electrically connected to a mounting-detecting part 92 of the slave control circuit 400. Whether the pair of terminals 91 are electrically conducted with each other is detected by the mounting-detecting part 92 which will be described below.

As shown in Figs. 19, 20, and 21, the regulating part 70C includes a support body 71C and a movable body 81C (an opening/closing member).

The support body 71C includes a column member 72C, an opening/closing motor 93, and a pinion 94.

The column member 72C is fixed to the base member 52. A through-hole part into which the movable body 81C is inserted is formed in the column member 72C. The through-hole part in the column member 72C supports the movable body 81C so that the movable body 81C is retractable into a movement path of the power source unit 56C moving along the guide rail 53, by a hole formed to have a central line extending in a direction orthogonal to the extending direction of the guide rail 53.

The column member 72C holds the movable body 81C at a boundary portion between the first region 54 and the second region 55 in the linear motion guide 51C.

As shown in Fig. 22, the opening/closing motor 93 is electrically connected to the slave control circuit 400. The opening/closing motor 93 rotates the pinion 94 (see Fig. 20) according to control, which will be described below, in the slave control circuit 400.

The pinion 94 shown in Fig. 20 is a spur gear fixed to an output shaft of the opening/closing motor 93 and connected to the movable body 81C.

The movable body 81C is a rod-shaped member which is advanceable and retractable in the through-hole part while being supported by the through-hole part of the column member 72C. A rack 95 extending in a longitudinal direction of the movable body 81C is formed at the movable body 81C. The rack 95 is a rack engaged with the pinion 94. The rack 95 is advanceable and retractable in the longitudinal direction thereof by rotation of the pinion 94. When the movable body 81C comes in contact with the substrate 57 of the power source unit 56C or the frame body 42 of the attachable/detachable part 41 (in the present embodiment, the movable body 81C comes in contact with the substrate 57 of the power source unit 56C), the movable body 81C regulates movement of the power source unit 56C along the guide rail 53. The movable body 81C of the present embodiment is disposed at a position at which the movable body 81C can come into contact with the power source unit 56C and does not come into contact with the attachable/detachable part 41.

The slave control circuit 400 shown in Fig. 22 controls a position of the movable body 81C of the regulating part 70C according to a position of the power source unit 56C on a movement path of the power source unit 56C along the guide rail 53 shown in Fig. 19 and a mounting state of the attachable/detachable part 41 on the power source unit 56C.

The slave control circuit 400 includes an opening/closing control part 401 configured to control a position of the movable body 81C of the regulating part 70C.

The opening/closing control part 401 includes the position-detecting part 87, the mounting-detecting part 92, and a driving control part 402.

The position-detecting part 87 determines that the power source unit 56C is disposed in the first region 54 on the guide rail 53 when the first sensing electrode 85 and the second sensing electrode 86 are in the insulating state. Also, the position-detecting part 87 determines that the power source unit 56C is disposed in the second region 55 on the guide rail 53 when the first sensing electrode 85 and the second sensing electrode 86 are in the conductive state.

The mounting-detecting part 92 determines that the substrate 57 and the frame body 42 are not engaged when the pair of terminals 91 in the switch 89 provided in the substrate 57 are in the insulating state. Also, the mounting-detecting part 92 determines that the substrate 57 and the frame body 42 are engaged when the pair of terminals 91 in the switch 89 provided in the substrate 57 are in the conductive state.

The driving control part 402 moves the movable body 81C according to a determined state in the position-detecting part 87 and a determined state in the mounting-detecting part 92.

Next, an example of a control flow in the opening/closing control part 401 will be described.

When the medical manipulator system 1C is in use, a position of the power source unit 56C and whether the attachable/detachable part 41 is mounted on the power source unit 56C are repeatedly detected.

As shown in Fig. 23, when conductivity of the pair of terminals 91 is detected by the mounting-detecting part 92 (YES in Step S1) and conductivity of the pair of sensing electrodes 84 is detected by the position-detecting part 87 (YES in Step S2), the driving control part 402 and the opening/closing motor 93 interpose the movable body 81C on the movement path of the power source unit 56C (Step S3).

As shown in Fig. 23, when conductivity of the pair of terminals 91 is detected by the mounting-detecting part 92 (YES in Step S1) and conductivity of the pair of sensing electrodes 84 is not detected by the position-detecting part 87 (NO in Step S2), the driving control part 402 and the opening/closing motor 93 move the movable body 81C out of the movement path of the power source unit 56C (Step S4).

As shown in Fig. 23, when conductivity of the pair of terminals 91 is not detected by the mounting-detecting part 92 (NO in Step S1), the movable body 81C is moved out of the movement path of the power source unit 56C by the driving control part 402 and the opening/closing motor 93.

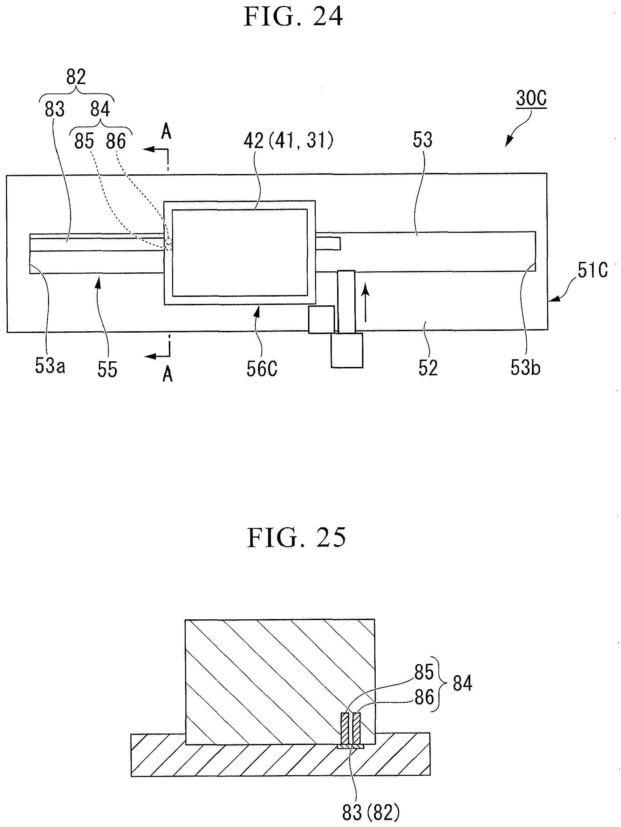

An operation of the movable body 81C in the medical manipulator system 1C according to the present embodiment will be described. Fig. 24 is a plan view showing a process in which a power source unit of a driver body moves along a guide rail of the driver body. Fig. 25 is a cross-sectional view taken along line A-A of Fig. 24.

In the present embodiment, when the position-detecting part 87 (see Fig. 22) determines that the power source unit 56C is in the first region 54 on the guide rail 53 as shown in Fig. 21, the driving control part 402 controls the opening/closing motor 93 provided in the support body 71C of the regulating part 70C to move the movable body 81C out of the movement path of the power source unit 56C along the guide rail 53 regardless of a determination in the mounting-detecting part 92.

When the power source unit 56C is disposed in the second region 55 on the guide rail 53 as shown in Fig. 24, the pair of sensing electrodes 84 are in a conductive state through the fixed electrode 83 as shown in Fig. 25. Accordingly, the driving control part 402 determines a position of the movable body 81C according to a determined state in the mounting-detecting part 92.

First, when the power source unit 56C is determined as being disposed in the second region 55 on the guide rail 53 and the substrate 57 and the frame body 42 are determined as being engaged with each other (see Fig. 24), the driving control part 402 controls the opening/closing motor 93 provided in the support body 71C of the regulating part 70C to insert the movable body 81C into the movement path of the power source unit 56C along the guide rail 53.

Second, when the power source unit 56C is determined as being disposed in the second region 55 on the guide rail 53, and the substrate 57 and the frame body 42 are determined as not being engaged with each other (i.e., when the attachable/detachable part 41 is detached from the power source unit 56C in the second region 55), the driving control part 402 controls the opening/closing motor 93 provided in the support body 71C of the regulating part 70C to move the movable body 81C out of the movement path of the power source unit 56C along the guide rail 53.

According to the present embodiment, the position sensor 82 configured to detect a position of the power source unit 56C of the present embodiment moving along the guide rail 53 by using the fixed electrode 83 provided at the linear motion guide 51C, the pair of sensing electrodes 84 provided in the power source unit 56, and the position-detecting part 87 provided in the slave control circuit 400 is configured.

The position sensor 82 of the present embodiment determines which of the first region 54 and the second region 55 of the guide rail 53 the power source unit 56C is disposed in on the basis of a contact state of the pair of sensing electrodes 84 to the fixed electrode 83. For example, because the first sensing electrode 85 and the second sensing electrode 86 are brought into the conductive state by the fixed electrode 83 when the power source unit 56C moves from the first region 54 to the second region 55, the position-detecting part 87 of the position sensor 82 determines that the power source unit 56C is disposed in the second region 55 on the guide rail 53.

The position sensor 82 is not limited to the above configuration. For example, the position sensor 82 may also include a known linear encoder.

The mounting sensor 88 configured to sense whether the attachable/detachable part 41 is mounted on the power source unit 56C by using the switch 89 provided in the substrate 57 of the power source unit 56C and the mounting-detecting part 92 provided in the slave control circuit 400 is configured.

The mounting sensor 88 is not limited to the above configuration. For example, the mounting sensor 88 may also include a known optical sensor.

Next, an operation and effect of the medical manipulator system 1C according to the present embodiment and an effect thereof will be described.

As shown in Fig. 21, when the power source unit 56C of the present embodiment is disposed in the first region 54, the movable body 81C is disposed out of the movement path of the power source unit 56C along the guide rail 53. Accordingly, the power source unit 56C is movable from the first region 54 to the second region 55 along the guide rail 53. In the present embodiment, the power source unit 56C is movable from the first region 54 to the second region 55 regardless of whether the attachable/detachable part 41 is attached to the power source unit 56C.

When the attachable/detachable part 41 is not attached to the power source unit 56C, because the pair of terminals 91 of the switch 89 provided in the substrate 57 are in the insulating state, the mounting-detecting part 92 determines that the substrate 57 and the frame body 42 are not engaged with each other. As a result, when the attachable/detachable part 41 is not attached to the power source unit 56C, a state in which the movable body 81C is disposed out of the movement path of the power source unit 56C along the guide rail 53 is maintained. Accordingly, when the attachable/detachable part 41 is not attached to the power source unit 56C, the power source unit 56C can be freely moved in any direction by passing through the boundary between the first region 54 and the second region 55.

When the attachable/detachable part 41 is attached to the power source unit 56C as shown in Fig. 21, since the pair of terminals 91 of the switch 89 provided in the substrate 57 are in the conductive state, the mounting-detecting part 92 determines that the substrate 57 and the frame body 42 are engaged with each other. When the power source unit 56C and the attachable/detachable part 41 integrally move from the first region 54 to the second region 55, since the first sensing electrode 85 and the second sensing electrode 86 are brought into the conductive state by the fixed electrode 83 as shown in Fig. 25, the position-detecting part 87 determines that the power source unit 56C is disposed in the second region 55 on the guide rail 53. As a result, the driving control part 402 controls the opening/closing motor 93 provided in the support body 71C of the regulating part 70C to insert the movable body 81C into the movement path of the power source unit 56C along the guide rail 53 as shown in Fig. 24. Accordingly, the power source unit 56C in the second region 55 cannot move to the first region 54.

When the attachable/detachable part 41 attached to the power source unit 56C in the second region 55 is, for example, detached from the power source unit 56C by the operator, the driving control part 402 controls the opening/closing motor 93 provided in the support body 71C of the regulating part 70C so that the movable body 81C is disposed outside the movement path of the power source unit 56C along the guide rail 53. Accordingly, after the attachable/detachable part 41 is detached from the power source unit 56C, the power source unit 56C can be moved from the second region 55 to the first region 54.

In the medical manipulator system 1C according to the present embodiment, a selective movement regulating means configured to selectively regulate movement of the power source unit 56C corresponding to a position of the power source unit 56C and an attachment state of the attachable/detachable part 41 to the power source unit 56C by using the regulating part 70C, the position sensor 82, the mounting sensor 88, and the opening/closing control part 401 is configured.

As described above, the medical manipulator system 1C according to the present embodiment also has the same effect as the medical manipulator system 1 disclosed in the first embodiment.

Further, according to the medical manipulator system 1C according to the present embodiment, because the movable body 81 is moved by the opening/closing motor 93, the movable body 81C can be prevented from being moved to an inappropriate position by a manual erroneous operation.

### (Third embodiment)

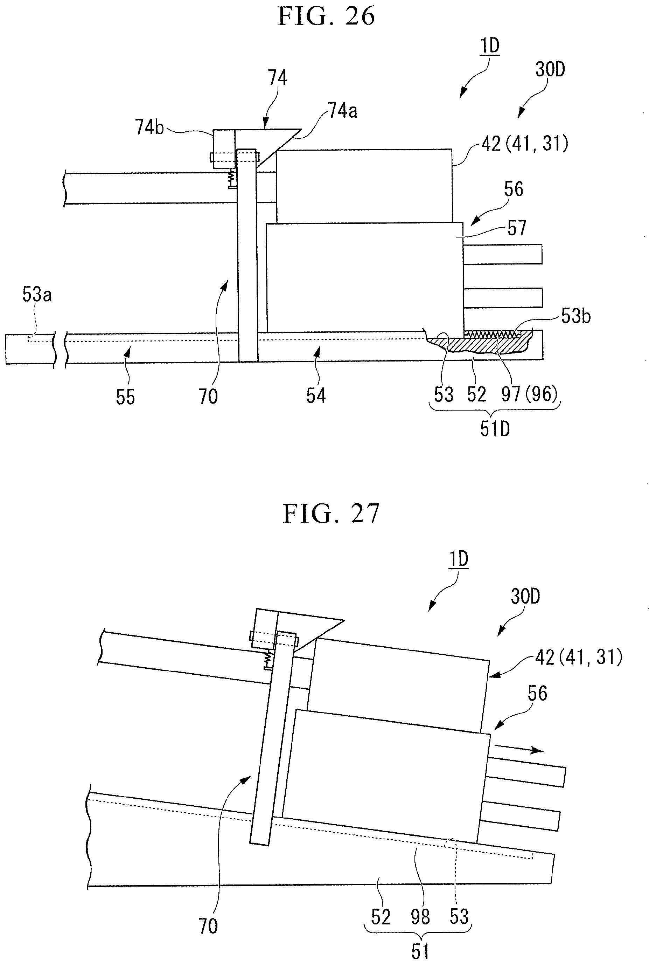

A third embodiment of the present invention will be described. Fig. 26 is a side view showing a treatment tool in a medical manipulator system according to a third embodiment of the present invention.

As shown in Fig. 26, a medical manipulator system 1D according to the present embodiment includes a treatment tool 30D having a different configuration with the treatment tool 30 disclosed in the first embodiment, instead of the treatment tool 30.

The treatment tool 30D of the present embodiment includes the insertion body 31 disclosed in the first embodiment and a driver body 50D provided instead of the driver body 50 disclosed in the first embodiment.

The driver body 50D includes the power source unit 56 and the regulating part 70 disclosed in the first embodiment and a linear motion guide 51D instead of the linear motion guide 51 disclosed in the first embodiment.

The linear motion guide 51D includes the base member 52 and the guide rail 53 disclosed in the first embodiment and a returning mechanism 96 configured to move the power source unit 56 toward the proximal end of the guide rail 53. When the power source unit 56 is disposed at the proximal end 53b of the guide rail 53, movement of the power source unit 56 is restricted by a stopper or the like (not shown) so that the power source unit 56 does not move further toward the proximal end 53b of the guide rail 53. In the state in which the power source unit 56 is disposed at the proximal end 53b of the guide rail 53, the treatment part 32 and the joint part 39 of the treatment tool 30 are disposed in the channel pipe 13 formed at the rigid distal end part 12 of the guide instrument 10. In the state in which the power source unit 56 is disposed at the proximal end 53b of the guide rail 53, a state of the power source 58 in a state corresponding to the joint part 39 which is linear in the channel pipe 13 can be stored as an initial state.

The returning mechanism 96 is formed of a towing spring 97 connected to the base member 52 or the guide rail 53 at the proximal end 53b of the guide rail 53 and connected to the power source unit 56. The towing spring 97 can be deformed through expansion and contraction in the extending direction of the guide rail 53 by the power source unit 56 moving along the guide rail 53.

An operation of the medical manipulator system 1D according to the present embodiment will be described.

In the present embodiment, the power source unit 56 is always towed by a constant force toward the proximal end 53b of the guide rail 53 by the towing spring 97 of the returning mechanism 96.

For example, when the power source unit 56 is in the second region 55 while integrally engaged with the attachable/detachable part 41 (see Fig. 13) and there is no force that attempts to move the power source unit 56 toward the distal end 53a of the guide rail 53, the power source unit 56 moves toward the proximal end 53b until the frame body 42 of the attachable/detachable part 41 comes into contact with the abutting surface 74b of the regulating part 70. The joint part 39 of the treatment tool 30 (see Fig. 10) is exposed from the distal end of the treatment tool channel 19.

When the attachable/detachable part 41 is detached from the power source unit 56 in the second region 55, by the action of the towing spring 97 which is the returning mechanism 96, the power source unit 56 passes through a portion between the rotator 74 and the guide rail 53 and moves up to the proximal end 53b of the guide rail 53.

As described above, according to the medical manipulator system 1D of the present embodiment, since the power source unit 56 is towed toward the proximal end 53b of the guide rail 53 by the returning mechanism 96, the attachable/detachable part 41 can be prevented from being mounted on the power source unit 56 at a side further toward the distal end past the proximal end 53b of the guide rail 53 by an erroneous operation.

When the power source unit 56 is in the first region 54, the power source unit 56 is towed by the towing spring 97 to be stopped at the proximal end 53b of the guide rail 53. Thus, in this state, when the attachable/detachable part 41 is connected to the power source unit 56 and the insertion body 31 is introduced into the treatment tool channel 19, the treatment part 32 and the joint part 39 are linearly disposed in the channel pipe 13 at the distal end part of the treatment tool channel 19. As a result, in the present embodiment, the treatment part 32 and the joint part 39 can be easily disposed in the channel pipe 13 for setting initial states of the treatment part 32 and the joint part 39.

### (Modified example 3-1)

Next, a modified example of the present embodiment will be described. Fig. 27 is a side view showing a configuration of the modified example of the present embodiment.

In the above-mentioned embodiment, an example is disclosed that the returning mechanism 96 is formed of the towing spring 97. In the present modified example, instead of the towing spring 97, the returning mechanism 96 includes a tilting table part 98 configured to hold the guide rail 53 by tilting the guide rail 53 with respect to the base member 52 so that the proximal end side of the guide rail 53 is below the distal end side thereof.

The tilting table part 98 which is the returning mechanism 96 is fixed to the base member 52 or integrally formed with the base member 52.

In the present modified example, when the base member 52 is horizontal as disclosed in the first embodiment, the guide rail 53 is supported by the tilting table part 98 so that the proximal end side of the guide rail 53 is below the distal end side thereof. Accordingly, the power source unit 56 which is movable along the guide rail 53 receives a force that attempts to move toward the proximal end 53b of the guide rail 53 by the gravitational action.

Such a configuration also has the same effect as the returning mechanism 96 of the above embodiment.

Instead of having the tilting table part 98 as the returning mechanism 96, by the slave control circuit 400 performing control in which the surgical power transmission adapter 220d (see Fig. 2) is tilted so that the proximal end side of the guide rail 53 is below the distal end side thereof, the power source unit 56 can be moved toward the proximal end 53b of the guide rail 53 by gravity by the same effect as the above embodiment.

### (Fourth embodiment)

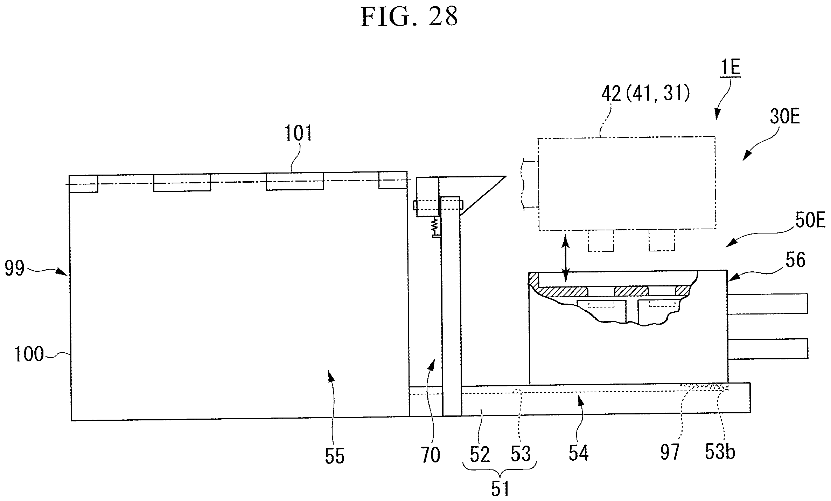

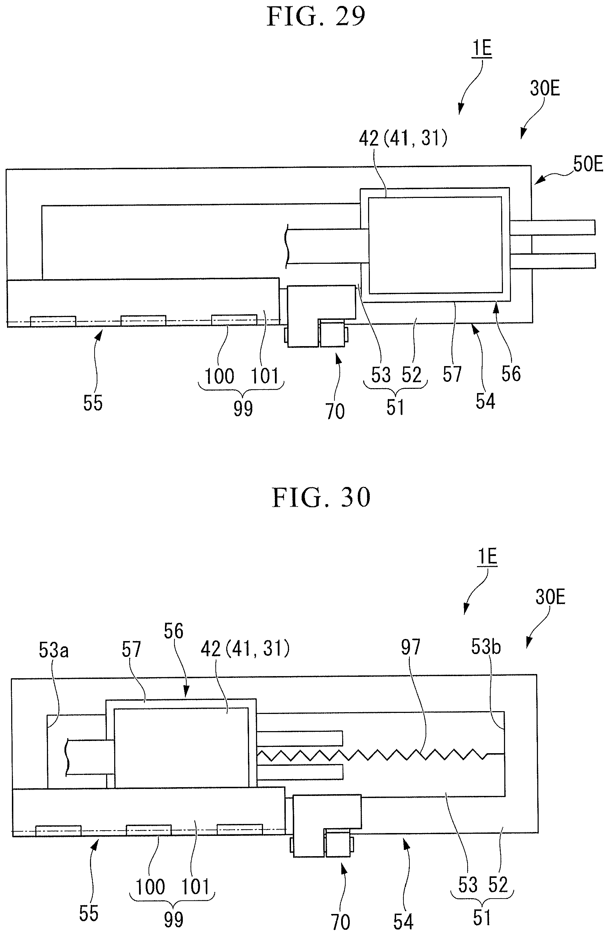

A fourth embodiment of the present invention will be described. Fig. 28 is a side view showing a treatment tool in a medical manipulator system according to the fourth embodiment of the present invention. Fig. 29 is a plan view of the treatment tool.

As shown in Figs. 28 and 29, a medical manipulator system 1E according to the present embodiment includes a treatment tool 30E having a configuration different from that of the treatment tool 30 disclosed in the first embodiment, instead of the treatment tool 30 disclosed in the first embodiment.

The treatment tool 30E of the present embodiment includes the insertion body 31 disclosed in the first embodiment and a driver body 50E provided instead of the driver body 50 disclosed in the first embodiment.

The driver body 50E includes the linear motion guide 51D, the power source unit 56, the regulating part 70 disclosed in the third embodiment, and a cover 99 (an attachment/detachment-regulating means) configured to partially surround the linear motion guide 51D, the power source unit 56, and the regulating part 70.

The cover 99 includes a wall part 100 formed at a portion of the linear motion guide 51 in the second region 55 and an opening/closing cover part 101 connected to the wall part 100.

The wall part 100 is attached to the base member 52 to openably and closably support the opening/closing cover part 101. The wall part 100 does not completely surround the linear motion guide 51 so that the operator can move the power source unit 56 along the guide rail 53.

The opening/closing cover part 101 is connected to the wall part 100 via a known hinge or the like. A spring (not shown) configured to bias the opening/closing cover part 101 in a closing direction of the opening/closing cover part 101 may be attached to the opening/closing cover part 101.

The opening/closing cover part 101 and the attachable/detachable part 41 do not come into contact in a state in which the opening/closing cover part 101 is closed. Accordingly, when the opening/closing cover part 101 is closed, the power source unit 56 and the attachable/detachable part 41 are integrally advanceable and retractable along the guide rail 53 in the second region 55.

An operation of the medical manipulator system 1E according to the present embodiment will be described. Fig. 30 is a side view showing a process in which a power source unit of a driver body moves along a guide rail of the driver body.

In the present embodiment, when the attachable/detachable part 41 is attached to or detached from the power source unit 56 in the first region 54 (a positional relationship shown in Fig. 29), the opening/closing cover part 101 does not need to be opened or closed. In contrast, when the attachable/detachable part 41 is attached to or detached from the power source unit 56 in the second region 55 (a positional relationship shown in Fig. 30), the attachable/detachable part 41 needs to be put in and taken out by opening the opening/closing cover part 101. When the attachable/detachable part 41 is not attached to the power source unit 56, the power source unit 56 is disposed in the first region 54 by the action of the towing spring 97.

In the present embodiment, when attachment of the attachable/detachable part 41 to the power source unit 56 is attempted in a state in which the attachable/detachable part 41 is not attached thereto, the attachable/detachable part 41 may be attached to the power source unit 56 in the first region 54 without taking time and effort such as moving the power source unit 56 to the second region 55 and further moving the opening/closing cover part 101.

In the present embodiment, since an attaching/detaching operation of the attachable/detachable part 41 in the second region 55 is intentionally made more complicated than an attaching/detaching operation of the attachable/detachable part 41 in the first region 54, mounting of the attachable/detachable part 41 in the first region 54 is made to be unconsciously selected by the operator instead of mounting of the attachable/detachable part 41 in the second region 55. In the present embodiment, since the power source unit 56 is stopped at the proximal end 53b of the guide rail 53 by the action of the towing spring 97, attachment of the attachable/detachable part 41 to the power source unit 56 is performed at the proximal end 53b of the guide rail 53.

When the attachable/detachable part 41 is attached to the power source unit 56, the operator may move the power source unit 56 and the attachable/detachable part 41 along the guide rail 53. Accordingly, it is possible to use the treatment tool 30 in the same manner as in the above embodiments.

In the state in which the attachable/detachable part 41 is attached to the power source unit 56, since the frame body 42 of the attachable/detachable part 41 abuts the abutting surface 74b of the rotator 74 as in the first embodiment, the power source unit 56 and the attachable/detachable part 41 cannot move to the first region 54. When the attachable/detachable part 41 is detached from the power source unit 56 after the treatment tool 30 is used, the attachable/detachable part 41 should be taken out by opening the opening/closing cover part 101 in the second region 55. When the attachable/detachable part 41 is detached from the power source unit 56, since there is no choice other than to take out the attachable/detachable part 41 by opening the opening/closing cover part 101, an erroneous operation is prevented.

According to the present embodiment, the cover 99 suppresses attachment of the insertion body 31 to the power source unit 56 disposed in the second region 55 and allows detachment of the insertion body 31 from the power source unit 56 when the insertion body 31 is already attached to the power source unit 56 disposed in the second region 55.

As described above, according to the medical manipulator system 1E of the present embodiment, by the cover 99 having the opening/closing cover part 101 being provided, the attachable/detachable part 41 can be prevented from being attached to the power source unit 56 in the second region 55 by an erroneous operation.

### (Fifth embodiment)

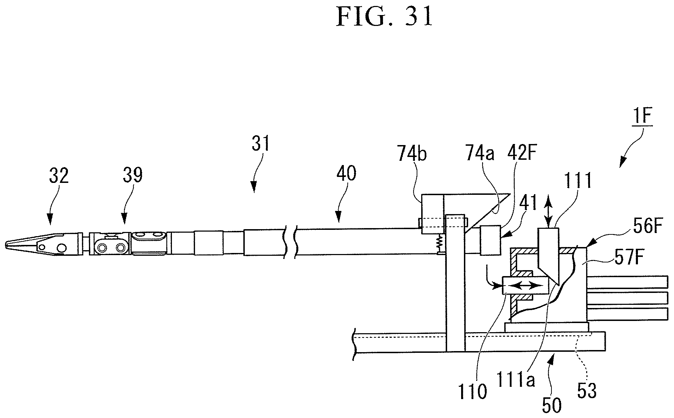

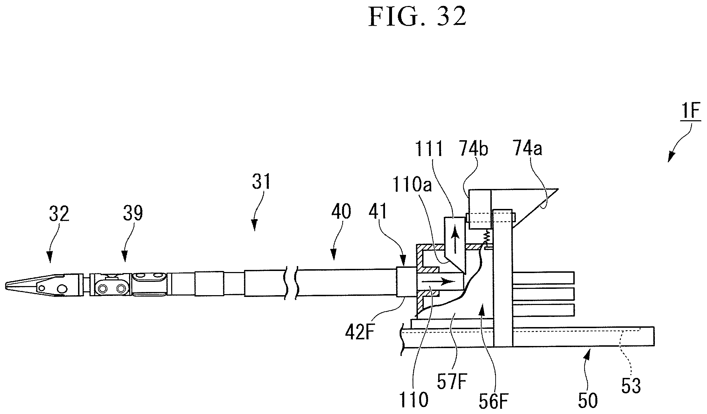

A fifth embodiment of the present embodiment will be described. Fig. 31 is a partial cross-sectional view showing a treatment tool in a medical manipulator system according to a fifth embodiment of the present invention in a side view. Fig. 32 is a view describing an action of the treatment tool.

As shown in Fig. 31, a medical manipulator system 1F according to the present embodiment includes a frame body 42F having a configuration different from that of the frame body 42 disclosed in the first embodiment, instead of the frame body 42 disclosed in the first embodiment. The medical manipulator system 1F according to the present embodiment includes a power source unit 56F having a configuration different from that of the power source unit 56 disclosed in the first embodiment, instead of the power source unit 56 disclosed in the first embodiment.

The frame body 42F is a member which is attachable to and detachable from a substrate 57F that is provided at the power source unit 56F and will be described below. For example, a hook structure (not shown) engaged with the substrate 57F of the power source unit 56F is formed in the frame body 42F. A configuration identical to the rotator part 43 disclosed in the first embodiment is provided in the frame body 42F.

The power source unit 56F includes a slide switch 110 disposed at a position at which the frame body 42F is attached, and a movable body 111 having a cam surface 111a which can come into contact with the slide switch 110. As the same with the first embodiment, the power source unit 56F of the present embodiment includes the substrate 57, the power source 58, and the power-transmitting part 62.

The slide switch 110 is a rod-shaped member which is advanceable and retractable in the extending direction of the guide rail 53. The slide switch 110 is projectable from and retractable into the substrate 57 in a state in which a part of the slide switch 110 is disposed inside the substrate 57. The slide switch 110 is pushed into the substrate 57 by an operation in which the frame body 42F is attached to the substrate 57.

The movable body 111 is a substantially rod-shaped member that is moved by the slide switch 110 when the slide switch 110 is pushed into the substrate 57. A surface of outer surfaces of the movable body 111 that comes into contact with the slide switch 110 is the cam surface 111 a tilted with respect to a moving direction of the slide switch 110. Accordingly, the movable body 111 can be moved since the cam surface 111a of the movable body 111 is pushed by a movement of the slide switch 110.

In the present embodiment, in the process in which the slide switch 110 is pushed into the substrate 57, the movable body 111 moves away from the guide rail 53 (see Fig. 32). In the state in which the slide switch 110 is pushed into the substrate 57, the movable body 111 can come into contact with the tapered surface 74a or the abutting surface 74b in the process in which the power source unit 56 moves along the guide rail 53.

In the present embodiment, since the movable body 111 provided at the power source unit 56F can come into contact with the tapered surface 74a and the abutting surface 74b instead of the frame body 42 coming into contact with the tapered surface 74a and the abutting surface 74b in the first embodiment, the present embodiment has the same effect as the first embodiment.

In the present embodiment, different with the first embodiment, it is not essential for the frame body 42F to come into contact with the tapered surface 74a and the abutting surface 74b.

In the present embodiment, optimizing a position or attitude of the treatment part 32 during treatment by rotating the treatment part 32 with the central line of the insertion body 31 as the center of rotation can be considered. For example, the frame body 42F and the substrate 57F may be integrally rotatable with the central line of the insertion body 31 as the center of rotation in a state in which the frame body 42F is attached to the substrate 57F. Also, in this case, the movable body 111 may radially protrude with respect to the central line of the insertion body 31 so that the movable body 111 can abut the tapered surface 74a and the abutting surface 74b even when the substrate 57F is rotated as above.

Although embodiments of the present invention have been described above with reference to the drawings, detailed configurations are not limited to the above embodiments, and design changes and the like within the scope not departing from the gist of the present invention are also included.

For example, in each of the embodiments described above, although a configuration in which a treatment tool is attached to a surgical power transmission adapter is exemplified, a driver body of the treatment tool may also be attached to an operating table or other tables.

Further, the treatment tool disclosed in each of the embodiments is also appropriately applicable to a surgical robot other than a master slave type manipulator system.

Further, the present invention is also appropriately applicable to a manual medical device not including power such as a motor.

Further, the elements shown in each of the above-described embodiments and modified examples may be configured by being appropriately combined.

### [Industrial Applicability]

The present invention can be used in a manipulator system which performs treatment on a treatment target by operating a treatment tool which is insertable into a treatment tool channel.

### [Reference Signs List]

1, 1A, 1B, 1C, 1D, 1E, 1F Medical manipulator system
10 Guide instrument
11 Inserting part
12 Rigid distal end part
13 Channel pipe
14 Imaging part
15 Active bending part
16 Flexible tube part
17 Channel tube
18 Treatment tool introduction opening
19 Treatment tool channel
20 Operation part
21 Case body
22 Angle knob
23 Dial part
24 Sprocket
25 Angle wire
30, 30A, 30B, 30C, 30D, 30E Treatment tool
31 Insertion body
32 Treatment part
33 Pair of jaws
34 First jaw
35 First grasping surface
36 Second jaw
37 Second grasping surface
38 Rotation shaft member
39 Joint part
40 Flexible part
41 Attachable/detachable part
42, 42F Frame body
43 Rotator part
44 Transmitting member
45 First transmitting member
46 Second transmitting member
50, 50A, 50B, 50C, 50D, 50E Driver body
51, 51C, 51D Linear motion guide
52 Base member
53 Guide rail
53b Proximal end of guide rail
54 First region
55 Second region
56, 56C, 56F Power source unit
57, 57F Substrate
58 Power source
59 Motor
60 First motor
61 Second motor
62 Power-transmitting part
70, 70A, 70B, 70C Regulating part
71, 71A, 71B, 71C Support
72, 72A, 72B, 72C Column member
73 Placing table part
73a Placing surface
74, 74A Rotator
74a, 74aB Tapered surface
74b, 74bB Abutting surface
75, 75A Connecting part
76, 76A Shaft member
77, 77A, 77B Biasing member
78 Regulating plate
78a Contact surface
79 Plate member
80 Holding part
81, 81C Movable body
81 a Contact surface
82 Position sensor
83 Fixed electrode
84 Sensing electrode
85 First sensing electrode
86 Second sensing electrode
87 Position-detecting part
88 Mounting sensor
89 Switch
90 Mover
91 Pair of terminals
92 Mounting-detecting part
93 Opening/closing motor
94 Pinion
95 Rack
96 Returning mechanism
98 Tilting table part
99 Cover
100 Wall part
101 Opening/closing cover part
110 Slide switch
111 Movable body
150 Operating table
200a to 200d Slave arm
220a, 220b, 220c, 220d Surgical power transmission adapter
240a to 240d Surgical tool
400 Slave control circuit
401 Opening/closing control part
402 Driving control part
500a, 500b Master arm
600 Operation part
700 Input-processing circuit
800 Image-processing circuit
900a Operator display
900b Assistant display

## Claims

1. A medical manipulator system, comprising:
a guide instrument that has a treatment tool channel and the guide instrument is inserted into a body;
an insertion body having a joint part that is bendable by receiving predetermined power and the insertion body is inserted into the treatment tool channel;
a power source unit attachable to and detachable from the insertion body and the power source unit is configured to transmit the power to the insertion body;
a guide rail having a first region which corresponds to a state in which the joint part is located in the treatment tool channel and a second region which is connected to the first region to correspond to a state in which the joint part is exposed from a distal end of the treatment tool channel, and the guide rail is configured to advance and retract the insertion body in the treatment tool channel by guiding the power source unit to advance and retract in a predetermined linear direction defined by the first region and the second region; and
a regulating part interposed in a boundary between the first region and the second region in an advancing/retracting path of the power source unit in the guide rail and the regulating part is configured to regulate a movement of the insertion body from the second region toward the first region in a state in which the insertion body and the power source unit are attached such that the power is able to be transmitted.

2. The medical manipulator system according to Claim 1,
wherein the regulating part includes:
an opening/closing member disposed on a movement path of the insertion body along the guide rail in a state in which the insertion body is attached to the power source unit to come into contact with at least part of the insertion body and the power source unit;
a support body configured to support the opening/closing member at the boundary such that the opening/closing member is able to be inserted into and removed from the movement path; and
a biasing member configured to bias the opening/closing member toward the support body such that the opening/closing member is disposed on the movement path,
wherein the opening/closing member includes:
a first surface facing the first region and the first surface is able to come into contact with at least part of the insertion body and the power source unit; and
a second surface facing the second region and the second surface is able to abut at least part of the insertion body and the power source unit in a direction of the movement path, and
wherein the insertion body comes into contact with the first surface to generate a space for introducing the insertion body into the second region by pushing the opening/closing member out of the movement path against a biasing force of the biasing member, in a state in which the insertion body is attached to the power source unit during a process in which the insertion body moves from the first region to the second region along the movement path.

3. The medical manipulator system according to Claim 2,
wherein the first surface is a tapered surface tilted with respect to the direction of the movement path; and
wherein at least part of the insertion body and the power source unit generates a space for introducing the insertion body into the second region by coming into contact with the tapered surface to push the opening/closing member out of the movement path against a biasing force of the biasing member, in a state in which the insertion body is attached to the power source unit during a process in which the insertion body moves from the first region to the second region.

4. The medical manipulator system according to Claim 2, wherein the opening/closing member is supported by the support body such that the opening/closing member is rotatable about a rotation axis extending in a direction orthogonal to the direction of the movement path.

5. The medical manipulator system according to Claim 1, further includes a returning mechanism configured to move the power source unit from the second region toward the first region along the guide rail.

6. The medical manipulator system according to Claim 1, further includes an attachment/detachment-regulating means configured to prevent an attachment of the insertion body to the power source unit disposed in the second region, and the attachment/detachment-regulating means is configured to allow a detachment of the insertion body from the power source unit when the power source unit disposed in the second region has already been attached to the insertion body.

7. The medical manipulator system according to Claim 1, further includes:
a mounting sensor capable of detecting that the insertion body is attached to the power source unit;
a position sensor capable of detecting which of the first region and the second region the power source unit is located at; and
a control unit configured to control the regulating part to regulate a movement of the power source unit between the first region and the second region, when the mounting sensor detects that the insertion body is attached to the power source unit and the position sensor detects that the power source unit is in the second region.

8. The medical manipulator system according to Claim 7, wherein the control unit cancels the control of the regulation by the regulating part and enables the power source unit to be movable from the second region to the first region, when the mounting sensor detects that the insertion body is not attached to the power source unit and the position sensor detects that the power source unit is in the second region.
